# EUROPEAN PATENT APPLICATION

(11) **EP 2 641 969 A1**
(43) Date of publication of application: **25.09.2013**
(21) Application number: 12160643.8
(22) Date of filing: 21.03.2012
(51) Int. Cl.: C12N 15/113, A61K 31/00, A61K 31/713

(54) **Novel therapeutic target for the prevention of tumour metastasis**

(71) Applicant: Max-Planck-Gesellschaft zur Förderung der Wissenschaften e.V., 80539 München (DE)
(72) Inventor: Offermanns, Stefan, 61231 Bad Nauheim (DE); Schumacher, Dagmar, 69120 Heidelberg (DE); Wettschureck, Nina, 61231 Bad Nauheim (DE); Strilic, Boris, 61231 Bad Nauheim (DE)
(74) Representative: Vossius & Partner

(57) **Abstract**

The present invention relates to an inhibitor of the P2Y2 receptor or an inhibitor of the P2Y2 receptor signaling pathway for use in preventing the metastasis of tumours or as a lead compound for developing a drug for preventing the metastasis of tumours.

## Description

The present invention relates to an inhibitor of the P2Y2 receptor or an inhibitor of the P2Y2 receptor signaling pathway for use in preventing the metastasis of tumours or as a lead compound for developing a drug for preventing the metastasis of tumours.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Tumour cell metastasis to distant organs is the primary cause of mortality in cancer patients. It is a complex and still incompletely understood process which requires tumour cells to leave the primary tumour, intravasate, survive in the circulation, extravasate by passing through the endothelial cell layer and grow in the target organ *(1-3)*. In various tumour models it has been demonstrated that pharmacological or genetic depletion of blood platelets strongly inhibits tumour cell metastasis *(4,5),* and it is now well-established that platelets are involved in tumour cell survival and dissemination *(6-9)*. Tumour cells can activate platelets through various mechanisms, and their metastatic potential correlates with their ability to activate platelets *(5, 8, 10-14).* The mechanisms by which platelets promote metastasis have remained elusive. Platelets may shield tumour cells against mechanical destruction by hemodynamic shear forces or against recognition and lysis by natural killer (NK) cells *(10, 15).* It is also possible that mitogenic stimuli or angiogenic factors released from tumour-activated platelets promote growth of tumour cells at secondary sites *(16-18)*. Most of these protein mediators are stored in platelet α-granules, while platelet dense granules contain small molecules such as ATP, ADP or serotonin *(19, 20).* Thus, the molecular mechanism underlying tumour metastasis are up to now unidentified despite ongoing and long-term research in this area.

The technical problem underlying the present invention was to identify alternative and/or improved means and methods to prevent tumour metastasis.

The solution to this technical problem is achieved by providing the embodiments characterized in the claims.

Accordingly, the present invention relates in a first embodiment to an inhibitor of the P2Y2 receptor or an inhibitor of the P2Y2 receptor signaling pathway for use in preventing the metastasis of tumours or as a lead compound for developing a drug for preventing the metastasis of tumours.

The term "P2Y2 receptor" is well-known in the art to relate to the P2Y purinoreceptor 2 which belongs to the family of G-protein coupled receptors (also abbreviated as P2Y₂ receptor). Said receptor is encoded by the P2RY2 gene in humans as a G-protein-coupled receptor with the seven transmembrane-spanning domains. There are three human transcript variants which encode for the same 377 amino acid protein sequence. The receptor has been reported to be expressed in brain (putamen striatum, caudate nucleus, hypothalamus, medulla oblongata, substantia nigra, thalamus, spinal cord), and other central nervous system structures as well as in smooth muscle cells, liver, testis, nasal epithelium and endothelial cells. The receptor couples to G-proteins of the G_{q}/G₁₁ family which mediate the stimulation of phosopholipase Cβ isoforms via the P2Y2 receptor. The following physiological functions have been reported for the receptor: presynaptic inhibition of glutamate release, positive inotropism, orientation of neutrophil chemotaxis, regulation of secretory responses in epithelia, regulation of migration of vascular smooth muscle cells. Natural ligands of the human P2Y2 receptor are in particular: ATP (EC₅₀ = 10^{-7.07}) and UTP (EC₅₀ = 10^{-8.1}), ADP activates the receptor with considerably lower potency. The P2RY2 gene encoding functionally active P2Y2 receptors has been described in many other mammalian species, such as for example including mouse, pig, rat, bovine, Chinese hamster or Mongolian gerbil. The amino acid sequence derived from the human P2Y2 cDNA sequence has been published in PNAS 91, 3275-3279 (1994) by E. Parr et al. The complete human protein and mRNA sequence can be retrieved from the database maintained online by the National Center for Biotechnology Information (NCBI), 8600 Rockville Pike, Bethesda MD, 20894 USA, using the accession number NP_788086 (protein sequence of transcript variant 1, version NP_788086.1, GI:28872745), Nip_002555 (protein sequence of transcript variant 2, version Nip_002555.2. GI:28872720) and NP_788085 (protein sequence of transcript variant 3, version NP_788085.1, GI:28872743), NM_176072 (mRNA of transcript variant 1, version NM_176072.1, GI:28872744), NM_002564 (mRNA of transcript variant 2, version NM_002564.2, GI:28872719), and NM_176071 (mRNA of transcript variant 3, version NM_176071.1, GI:28872742). Homologous P2Y2 nucleic acid and amino acid sequences of other species can equally be retrieved in publicly available databases, such as, e.g., that mentioned above.

The term "inhibitor" in accordance with the present invention refers to an inhibitor that reduces or abolishes the biological function or activity of the P2Y2 receptor or a target that is part of the P2Y2 receptor signaling pathway. An inhibitor may perform any one or more of the following effects in order to reduce or abolish the biological function or activity of the protein to be inhibited: (i) the transcription of the gene encoding the protein to be inhibited is lowered, i.e. the level of mRNA is lowered, (ii) the translation of the mRNA encoding the protein to be inhibited is lowered, (iii) the protein performs its biochemical function with lowered efficiency in the presence of the inhibitor, and (iv) the protein performs its cellular function with lowered efficiency in the presence of the inhibitor.

Compounds falling in class (i) include compounds interfering with the transcriptional machinery and/or its interaction with the promoter of said gene and/or with expression control elements remote from the promoter such as enhancers. Compounds of class (ii) comprise antisense constructs and constructs for performing RNA interference (e.g. siRNA) well known in the art (see, e.g. Zamore (2001) Nat Struct Biol. 8(9), 746; Tuschl (2001) Chembiochem. 2(4), 239). Compounds of class (iii) interfere with molecular function of the protein to be inhibited, such as receptor signaling activity and activation of downstream target molecules. Accordingly, active site binding compounds are envisaged. Class (iv) includes compounds which do not necessarily bind directly to the target, but still interfere with its function or activity, for example by binding to and/or inhibiting the function or inhibiting expression of members of a pathway which comprises the target. These members may be either upstream or downstream of the target within said pathway. For example, such compounds may alter the affinity or rate of binding of a known ligand to the receptor, compete with a ligand for binding to the receptor, displace a ligand bound to the receptor or reduce the amount of ligand available for binding.

The inhibitor, in accordance with the present invention, may in certain embodiments be provided as a proteinaceous compound or as a nucleic acid molecule encoding the inhibitor. For example, the nucleic acid molecule encoding the inhibitor may be incorporated into an expression vector comprising regulatory elements, such as for example specific promoters, and thus can be delivered into a cell. Methods for targeted transfection of cells and suitable vectors are known in the art, see for example Sambrook and Russel ("Molecular Cloning, A Laboratory Manual", Cold Spring Harbor Laboratory, N.Y. (2001)). Incorporation of the nucleic acid molecule encoding the inhibitor into an expression vector allows to either selectively or permanently elevate the level of the encoded inhibitor in any cell or a subset of selected cells of a recipient. Thus, a tissue- and/or time-dependent expression of the inhibitor can be achieved, for example restricted to endothelial cells. In a preferred embodiment, the inhibitor is therefore a endothelial cell-specific inhibitor.

The term "inhibitor of the P2Y2 receptor" in accordance with the present invention refers to an inhibitor that reduces the biological function or activity of the P2Y2 receptor. Biological function or activity denotes in particular any known biological function or activity of the P2Y2 receptor including those elucidated in accordance with the present invention. Examples of said biological function or activity are the activation of the P2Y2 signaling pathway, including its capability of recruiting G-proteins G_{q}/G₁₁, activation of protein kinase C (PKC), elevation of intracellular Ca²⁺-levels, thereby resulting in the reduction of endothelial barrier function, orientation of neutrophil chemotaxis (Y. Chen et al., Science, 314, 1792-1795 (2006)) or regulation of migration of vascular smooth muscle cells. (N. Yu et al., Circ Res, 102, 581-588 (2008)) as well as those mentioned herein in other sections (cf., e.g., the example section). All these functions or activities can be tested for either using any of a variety of standard methods known in the art, such as *in vitro* impedance measurements on an endothelial cell monolayer or *in vivo* measurements of extravasation (e.g. of Evans blue) (see H.Korhonen et al., J. Exp. Med. 206, 411 (2009)) for assessing the barrier function, determination of receptor-dependent activation of GPTγS binding or receptor-dependent stimulation of GTPase activity or receptor binding of other nucleotides to G-proteins *in vitro* for assessing coupling of G-proteins G_{q}/G₁₁ to the P2Y2 receptor, determination of β-arrestin binding to P2Y2 and P2Y2 receptor internalization for assessing receptor activation independently of hetertrimeric G-proteins, or on the basis of the teachings of the examples provided below, optionally in conjunction with molecular techniques such as RT-PCR or with the teachings of the documents cited therein.

In a preferred embodiment, the inhibitor reduces the biological function or activity of the P2Y2 receptor by at least 50%, preferably by at least 75%, more preferred by at least 90% and even more preferred by at least 95% such as at least 98% or even by 100% as compared to the biological function or activity in the absence of said inhibitor. The term reduction by at least, for example 75%, refers to a decreased biological function or activity such that the P2Y2 receptor looses 75% of its function or activity and, consequently, has only 25% of the function or activity remaining as compared to a P2Y2 receptor that is not inhibited. Also preferred, biological function or activity of the P2Y2 receptor drops to less than 10⁻², less than 10⁻³, less than 10⁻⁴ or less than 10⁻⁵ times the biological function or activity compared to the biological function or activity in the absence of said inhibitor. As outlined herein above, the reduction of the biological function or activity of the P2Y2 receptor is mediated by inhibitors using different mechanisms of actions. Depending on said mechanism of action, a reduction of, e.g., 75% may be achievable by a given inhibitor by reducing the biological function or activity of all or substantially all of the P2Y2 receptors by 75% or by fully inhibiting 75% of all or substantially all of the P2Y2 receptors. In other words, the reduction of said biological function or activity may be of qualitative or quantitative nature. The term "substantially all" is meant to specify that at least 95% or more of the P2Y2 receptors are encompassed. The use of the term "substantially all" is a tribute to the constant changes of protein expression observed in a cell that may prevent truly addressing all of the P2Y2 receptors.

The function of any of the inhibitors referred to in the present invention may be identified and/or verified by using, e.g., high throughput screening assays (HTS). High-throughput assays, independently of being biochemical, cellular or other assays, generally may be performed in wells of microtiter plates, wherein each plate may contain, for example 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits biological activity, said mixture of test compounds may be de-convoluted to identify the one or more test compounds in said mixture giving rise to the observed biological activity.

The determination of the binding of potential inhibitors can be effected in, for example and without limitation, any binding assay, such as a biophysical binding assay, which may be used to identify binding of test molecules prior to performing the functional/activity assay with the inhibitor. Suitable biophysical binding assays are known in the art and comprise fluorescence polarization (FP) assay, fluorescence resonance energy transfer (FRET) assay and surface plasmon resonance (SPR) assay. Instead of or in addition to assessing the direct interaction of inhibitor and target molecule by binding assays, one may indirectly determine the interaction of the inhibitor with its target molecule by using a suitable read out. For example, in cases where the inhibitor acts by decreasing the expression level of the target protein, the determination of the expression level of the protein can, for example, be carried out on the nucleic acid level or on the amino acid level.

Methods for determining the expression of a protein on the nucleic acid level include, but are not limited to, northern blotting, PCR, RT-PCR or real RT-PCR.

A northern blot allows the determination of RNA or isolated mRNA in a sample. Northern blotting involves the use of electrophoresis to separate RNA samples by size and detection with a hybridization probe complementary to part of or the entire target sequence. Initially, total RNA extraction from the sample is performed. If desired, mRNA can be separated from said initial RNA sample through the use of oligo (dT) cellulose chromatography to isolate only the RNA with a poly(A) tail. RNA samples are then separated by gel electrophoresis. The separated RNA is then transferred to a nylon membrane through a capillary or vacuum blotting system. After transfer to the membrane, the RNA is immobilized through covalent linkage to the membrane by, e.g., UV light or heat. Then, the RNA is detected using suitably labeled probes and X-ray film and can subsequently be quantified by densitometry. Suitable compositions of gels, buffers and labels are well-known in the art and may vary depending on the specific sample and target to be identified.

PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed, for example, in a 50 µl reaction mixture containing 5 µl of 10 x PCR buffer with 1.5 mM MgCI₂, 200 µM of each deoxynucleoside triphosphate, 0.5 µl of each primer (10 µM), about 10 to 100ng of template DNA and 1 to 2.5 units of Taq polymerase. The primers for the amplification may be labeled or be unlabeled. DNA amplification can be performed, e.g., with a Applied Biosystems Verlti® Thermal Cycler (Life Technologies Corporation, Carlsbad, CA), C1000™ thermal cycler (Bio-Rad Laboratories, Hercules, CA,), or SureCycler 8800 (Agilent Technologies, Santa Clara, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step, e.g. at 55°C for 1 min as well as a final extension step, e.g. at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus Vent, Amplitaq, Pfu and KOD, some of which may exhibit proof-reading function and/or different temperature optima. However, it is well known in the art how to optimize PCR conditions for the amplification of specific nucleic acid molecules with primers of different length and/or composition or to scale down or increase the volume of the reaction mix. The "reverse transcriptase polymerase chain reaction" (RT-PCR) is used when the nucleic acid to be amplified consists of RNA. The term "reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and *Thermus thermophilus* DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. High-temperature RT provides greater primer specificity and improved efficiency. U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. *Thermus thermophilus* DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT reaction can be performed, for example, in a 20µl reaction mix containing: 4 µl of 5x AMV-RT buffer, 2 µl of oligo dT (100 µg/ml), 2µl of 10 mM dNTPs, 1µl total RNA, 10 Units of AMV reverse transcriptase, and H₂O to 20µl final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample. Any of the above mentioned reaction conditions may be scaled up according to the needs of the particular case. The resulting products are loaded onto an agarose gel and band intensities are compared after staining the nucleic acid molecules with an intercalating dye such as ethidium bromide or SybrGreen. A lower band intensity of the sample treated with the inhibitor as compared to a non-treated sample indicates that the inhibitor successfully inhibits the protein.

Real-time PCR employs a specific probe, in the art also referred to as TaqMan probe, which has a reporter dye covalently attached at the 5' end and a quencher at the 3' end. After the TaqMan probe has been hybridized in the annealing step of the PCR reaction to the complementary site of the polynucleotide being amplified, the 5' fluorophore is cleaved by the 5' nuclease activity of Taq polymerase in the extension phase of the PCR reaction. This enhances the fluorescence of the 5' donor, which was formerly quenched due to the close proximity to the 3' acceptor in the TaqMan probe sequence. Thereby, the process of amplification can be monitored directly and in real time, which permits a significantly more precise determination of expression levels than conventional endpoint PCR. Also of use in real time RT-PCR experiments is a DNA intercalating dye such as SybrGreen for monitoring the de novo synthesis of double stranded DNA molecules.

Methods for the determination of the expression of a protein on the amino acid level include, but are not limited to, western blotting or polyacrylamide gel electrophoresis in conjunction with protein staining techniques such as Coomassie Brilliant blue or silver-staining. The total protein is loaded onto a polyacrylamide gel and electrophoresed. Afterwards, the separated proteins are transferred onto a membrane, e.g. a polyvinyldifluoride (PVDF) membrane, by applying an electrical current. The proteins on the membrane are exposed to an antibody specifically recognizing the protein of interest. After washing, a second antibody specifically recognizing the first antibody and carrying a readout system such as a fluorescent dye is applied. The amount of the protein of interest is determined by comparing the fluorescence intensity of the protein derived from the sample treated with the inhibitor and the protein derived from a non-treated sample. A lower fluorescence intensity of the protein derived from the sample treated with the inhibitor indicates a successful inhibitor of the protein. Also of use in protein quantification is the Agilent Bioanalyzer technique (e.g., Agilent 2100 Bioanalyzer; Agilent Technologies, Santa Clara, CA).

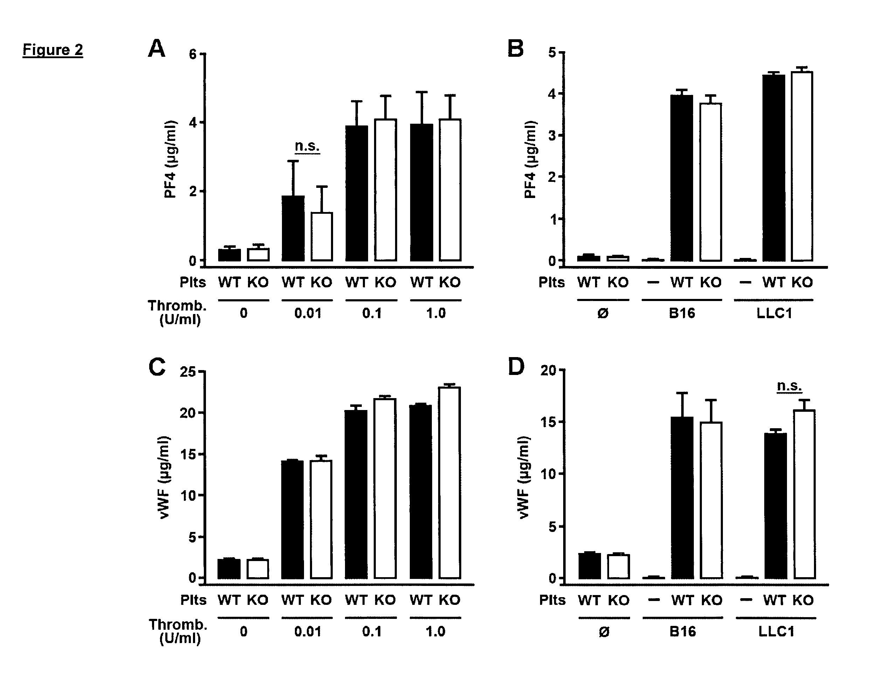

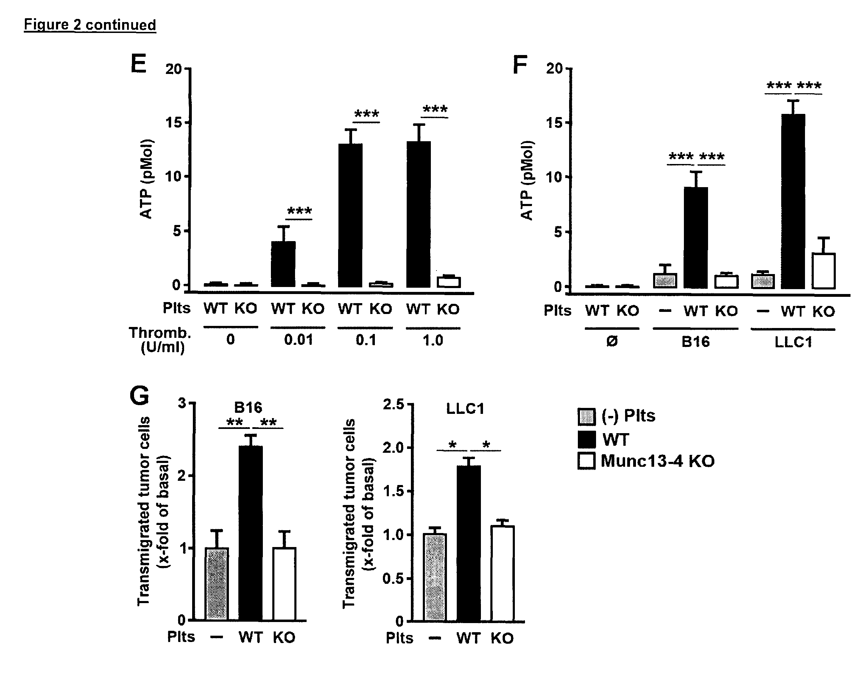

The term "inhibitor of the P2Y2 receptor signaling pathway" as used in accordance with the present invention refers to any inhibitor that does not directly interact with the P2Y2 receptor but with target molecules that directly or indirectly are involved in the activation of one or more of the biological functions or activities of the P2Y2 receptor and preferably one or more of those functions or activities referred to above or elsewhere in this specification, wherein the molecules are situated upstream or downstream within the P2Y2 receptor signaling pathway. Specifically, and in line with the description of the P2Y2 receptor signaling pathway given herein above, an inhibitor of the P2Y2 signaling pathway may inhibit target molecules downstream of the P2Y2 receptor or upstream. As exemplary downstream target molecules of the P2Y2 signaling pathway a suitable inhibitor can inhibit the binding of the G-proteins G_{q}/G₁₁ to the P2Y2 receptor, or can inhibit the activity of the protein kinase C (PKC). As exemplary targets upstream in the P2Y2 receptor signaling pathway the prominent ligand of the receptor, i.e. ATP, can be chosen. While ADP is also a ligand of the P2Y2 receptor and is being secreted from platelets, it is a less effective agonist of P2Y2 receptors in comparison to ATP but can basically activate the receptor (K. D. Lustig et al., Proc. Natl. Acad. Sci. U. S A. 90, 5113-5117 (1993); E. R. Lazarowski et al., Br. J. Pharmacol. 116, 1619-1627 (1995); C. E. Parr et al., PNAS 91, 3275-3279 (1994)). UTP, which is a full agonist of P2Y2, comparable to ATP, is, however, only released in small quantities from activated platelets. Therefore, in accordance with the invention, preferably ATP is chosen if the target upstream from the P2Y2 receptor has been determined to be a ligand. Also, molecules involved in mediating the release of ATP stored in platelet dense granules, preferably the vesicle priming factor Munc13-4, can be chosen. Munc13 proteins are considered to be the major priming proteins for new transmitter release from neurons, however, they also play important roles in vesicle release in other non-neuronal cells, including platelets (I. Ausgustin et al., Nature 400, 457-461 (1999); F, Varoqueaux et al., Proc. Natl. Acad. Sci. U. S. A. 99, 9037-9042 (2002); Q. Ren et al., Blood 116, 869-877 (2010); K. Crozat et al., J. Exp. Med. 204, 853-863 (2007)). In particular the Munc-13-4 isoform has been shown to be specifically expressed by megakaryocytes, the precursors of platelets, and Munc-13-4 is shown in the literature to be involved in platelet granule secretion (Q. Ren et al., Blood 116, 869-877 (2010); K. Crozat et al., J. Exp. Med. 204, 853-863 (2007)). The advantage of Munc13-4 compared to other components of the exocytotic machinery of platelets as target to interfere in platelet dense granule secretion is as follows: no individual SNARE (Soluble NSF Attachment Protein Receptor) protein has been found to be solely responsible for exocytosis in platelets since there is obviously a strong functional redundancy among SNARE proteins in exocytotic function. In contrast, Munc-13-4 deletion in platelets completely blocks dense granule secretion but has no effect on α-granule secretion (see also Fig. 2). Preferably, the inhibitor of the P2Y2 receptor signaling pathway targets a molecule downstream of the P2Y2 receptor within the P2Y2 receptor signaling pathway.
The inhibitor classification and the inhibition values referred to above for the inhibitor of the P2Y2 receptor apply *mutatis mutandis* to the inhibitor of the P2Y2 receptor signaling pathway. In case, the inhibitor targets not a protein, such as in the case of ATP as a target, the inhibitor inhibits the biological function or activity of said target by, e.g., inhibiting its binding to binding partners and/or by diminishing its concentration.

For example, the inhibitor of the P2Y2 receptor signaling pathway may be any compound that reduces the amount of the G-proteins G_{q}/G₁₁ available for binding to the P2Y2 receptor. Such a compound may act on G_{q}/G₁₁ directly, such as for example by reducing its expression levels or its binding abilities to the P2Y2 receptor or it may reduce the level of GTP bound by the G-protein thereby preventing G_{q}/G₁₁ activation. Thus, also encompassed by the term inhibitor of the P2Y2 receptor signaling pathway are compounds that reduce the level of GTP in a cell, preferably an endothelial cell so as to achieve the prevention of tumour metastasis.

It is understood by the person skilled in the art that the biological activity or function of the P2Y2 receptor which renders it suitable as therapeutic target for the prevention of tumour metastasis can be inhibited by one or more inhibitor(s), as described herein above, by directly acting on the P2Y2 receptor or indirectly by inhibiting downstream or upstream members of the P2Y2 receptor signaling pathway, preferably provided that the different inhibitors do not interfere with one another which can be tested in accordance with methods known in the art and/or described herein. It is preferred that the inhibitors provide an additive effect and, optionally, a synergistic effect in their inhibitory activity. The P2Y2 receptor is part of a signaling pathway (as described in detail above) thus making it possible to target molecules being part of said pathway upstream and/or downstream of the P2Y2 receptor to achieve inhibition of the biological activity or function attributed to the P2Y2 receptor described herein and known in the art. Preferably, an inhibitor in accordance with the invention directly or indirectly, but specifically inhibits the biological activity or function of the P2Y2 receptor and/or G_{q}/G₁₁. The term "specifically" in this context refers to the capability of an inhibitor to not have an effect or an essential effect on other molecules than the target molecules. In other words, a corresponding inhibitor does not display cross-reactivity or essentially does not display cross-reactivity. In this context, the term "essentially" is meant to refer to an insignificant or negligible effect. The insignificance or negligibility can be based on functional or quantitative parameters. For example, only a minimal amount of cross-reactivity occurs with a different non-target molecule with a negligible or insignificant effect, and/or cross-reactivity occurs, however, with a non-target molecule that is present in insignificant amounts and/or the binding of the inhibitor to the non-target molecule is of no consequence.

Preferably, the inhibitor of the present invention is comprised in a pharmaceutical composition, preferably further comprising a pharmaceutically acceptable carrier, excipient and/or diluent.

The term "pharmaceutical composition", as used herein, relates to a composition for administration to a patient, preferably a human patient. The pharmaceutical composition of the invention comprises at least one, such as at least two, e.g. at least three, in further embodiments at least four such as at last five of the inhibitors mentioned herein. The invention also envisages mixtures of inhibitors of the P2Y2 receptor or of inhibitors of the P2Y2 receptor signaling pathway. In cases where more than one inhibitor is comprised in the pharmaceutical composition it is understood that none of these inhibitors has any or any essentially inhibitory effect on the other inhibitors also comprised in the composition. The term "essentially" in this context refers to an insignificant or negligible inhibitory effect. Again, it is preferred that the inhibitors provide an additive effect and, optionally, a synergistic effect in their inhibitory activity.

The composition may be in solid, liquid or gaseous form and may be, inter alia, in a form of (a) powder(s), (a) tablet(s), (a) solution(s) or (an) aerosol(s).

As mentioned above, it is preferred that said pharmaceutical composition comprises a pharmaceutically acceptable carrier, excipient and/or diluent. Examples of suitable pharmaceutical carriers, excipients and/or diluents are well known in the art and include phosphate buffered saline solutions, water, emulsions, such as oil/water emulsions, various types of wetting agents, sterile solutions etc. The carriers, excipients and diluents to some extent depend on the chemical nature of the actual inhibitors and can be chosen by the skilled person according to established protocols. Compositions comprising such carriers can be formulated by well known conventional methods. These pharmaceutical compositions can be administered to the subject at a suitable dose. Administration of the suitable compositions may be effected by different ways, e.g., by intravenous, oral, rectal, intraperitoneal, subcutaneous, intramuscular, topical, intradermal, intranasal or intrabronchial administration. It is preferred that the mode of administration is a systemic administration such as, e.g., carried out by injection and/or delivery, e.g., to a site in the bloodstream such as a coronary artery. The compositions of the invention may also be administered directly to the target site, e.g., by biolistic delivery to an external or internal target site, preferably the site where the tumour is located. Only in cases where the region of metastasis or the organ to which tumour cells metastasize is small and defined, localized administration may be preferred over systemic administration. In theses cases, local administration may have be advantageous to, e.g., minimize the amount of drug used or decrease the risk of adverse side effects, if any. A combination of systemic and localized administration may be chosen in order to achieve a temporally increased concentration of the inhibitor as defined herein at a specific site in the body. The dosage regimen will be determined by the attending physician and clinical factors. As is well known in the medical arts, dosages for any one patient depend upon many factors, including the patient's size, body surface area, age, the potency and bioavailability of the particular compound to be administered, sex, time and route of administration, general health, and other drugs being administered concurrently. In particular, the potency and mode of action of an inhibitor may dictate not only its dosage, but also its way of administration. For example, inhibitors that due to their mode of action completely abolish the biological activity or function of a P2Y2 receptor (or a target molecule within the P2Y2 receptor signaling pathway) when binding to the latter, may potentially not be suitable for systemic administration due to the possible occurrence of unwanted side effects, if parameters such as, e.g., bioavailability cannot be sufficiently controlled in the sense of minimizing given unwanted side effects. Pharmaceutically active matter may be present in amounts between 1 ng and 10 mg/kg body weight per dose; however, doses below or above this exemplary range are envisioned, especially considering the aforementioned factors. If the regimen is a continuous infusion, it should also be in the range of 0.01 µg to 10 mg units per kilogram of body weight per minute. The continuous infusion regimen may be completed with a loading dose in the dose range of 1 ng and 10 mg/kg body weight.

In the case of localized delivery of the inhibitors of the present invention to the site of the tumour, various options exist to achieve said site-specific delivery to the tumour(s). For example, the inhibitor(s) may be functionalized in that moieties are attached that specifically target tumour cells. Based on the specific expression of defined antigens by tumour cells, inhibitors may be attached to antibodies or antibody fragments specifically interacting with the antigen expressed by tumour cells. Alternatively, the vehicle carrying the inhibitor(s) may be functionalized so that specifically tumour cells are targeted. In cases where tumour cells exhibit particular enzymatic activities or uptake mechanisms, inhibitor can be functionalized in a way that it is converted by the enzymatic activity into a functionally active form or that it is subject to the specific uptake mechanism, respectively.

Progress can be monitored by periodic assessment. The compositions of the invention may be administered locally or systemically. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. It is particularly preferred that said pharmaceutical composition comprises further agents known in the art to antagonize heart failure. Since the pharmaceutical preparation of the present invention relies on the above mentioned inhibitors, it is preferred that those mentioned further agents are only used as a supplement, i.e. at a reduced dose as compared to the recommended dose when used as the only drug, so as to e.g. reduce side effects conferred by the further agents. Conventional excipients include binding agents, fillers, lubricants and wetting agents.

The term "metastasis" in the context of tumours is well-known in the art. The term "tumour" as used herein relates to neoplasms, e.g. characterized by abnormal tissue proliferation, that are known to or are suspected to metastasize and thus warrant the use of inhibitors as defined herein to prevent metastasis. Tumours that are known to metastasize are malignant tumours (also referred to in the art as cancerous tumours) in contrast to benign tumours that lack the ability to form metastases. However, many types of the latter tumours have the potential to become malignant, i.e. metastasize, (such as, e.g., teratoma). Thus, this kind of tumours are suspected to metastasize and therefore also benign tumours may in certain cases warrant the use of the inhibitors as defined herein to prevent metastasis. Tumours in accordance with the invention are i) carcinoma, ii) sarcoma, iii) carcinosarcoma, iv) tumours of the blood-forming tissues, v) tumours of the nerve tissue including the brain, and vi) cancer of skin cells. A tumour according to i) occurs in epithelial tissues, which cover the outer body (the skin) and line mucous membranes and the inner cavitary structures of organs e.g. such as the breast, lung, the respiratory and gastrointestinal tracts, the endocrine glands, and the genitourinary system. Ductal or glandular elements may persist in epithelial tumours, as in adenocarcinomas like, e.g., thyroid adenocarcinoma, gastric adenocarcinoma, uterine adenocarcinoma. Tumours of the pavement-cell epithelium of the skin and of certain mucous membranes, such as e. g. tumours of the tongue, lip, larynx, urinary bladder, uterine cervix, or penis, may be termed epidermoid or squamous-cell carcinomas of the respective tissues and are in the scope of the definition of tumour as well. A tumour according to ii) develops in connective tissues, including fibrous tissues, adipose (fat) tissues, muscle, blood ves sels, bone, and cartilage like e.g. osteogenic sarcoma, liposarcoma, fibrosarcoma, or synovial sarcoma. A tumour according to iii) is a tumour that develops in both epithelial and connective tissue. A tumour within the scope of this definition may be a primary or secondary tumour, whereby primary indicates that the tumour originated in the tissue where it is found rather than was established as a secondary site through metastasis from another tumour lesion. As outlined above, tumours within the scope of this definition may be benign or malign and may affect all anatomical structures of the body of a subject, preferably a mammal. For example, they comprise tumours of a) the bone marrow and bone marrow derived cells (leukemias), b) the endocrine and exocrine glands like e. g. thyroid, parathyroid, pituitary, adrenal glands, salivary glands, pancreas, c) the breast, like e. g. benign or malignant tumours in the mammary glands of either a male or a female, the mammary ducts, adenocarcinoma, medullary carcinoma, comedo carcinoma, Paget's disease of the nipple, inflammatory carcinoma of the young woman, d) the lung, e) the stomach, f) the liver and spleen, g) the small intestine, h) the colon, i) the bone and its supportive and connective tissues like malignant or benign bone tumour, e. g. malignant osteogenic sarcoma, benign osteoma, cartilage tumors; like malignant chondrosarcoma or benign chondroma; bone marrow tumours like malignant myeloma or benign eosinophilic granuloma, as well as metastatic tumors from bone tissues at other locations of the body; k) the mouth, throat, larynx, and the esophagus, 1) the urinary bladder and the internal and external organs and structures of the urogenital system of male and female like ovaries, uterus, cervix of the uterus, testes, and prostate gland, m) the prostate, n) the pancreas, like ductal carcinoma of the pancreas; o) the lymphatic tissue like lymphomas and other tumors of lymphoid origin, p) the skin, q) cancers and tumor diseases of all anatomical structures belonging to the respiration and respiratory systems including thoracal muscles and linings, r) primary or secondary cancer of the lymph nodes s) the tongue and of the bony structures of the hard palate or sinuses, t) the mouth, cheeks, neck and salivary glands, u) the blood vessels including the heart and their linings, v) the smooth or skeletal muscles and their ligaments and linings, w) the peripheral, the autonomous, the central nervous system including the cerebellum, x) the adipose tissue. The tumours may be in any stage of their development such as early stages, where the tumour is still confined to the tissue of origin (or primary site), or when the cancer cells have already invaded adjacent tissue or distant parts of the body from the primary site, i.e. the tumour already metastasizes. Metastasis of tumors is the process in which cancer cells can detach from the primary tumor and produce daughter tumors either locally or after spreading via blood or lymphatic vessels. Tumors which have a high incidence of metastasis are e.g. tumors of the lung, breast, colon, kidney, prostate, pancreas, cervix, as well as melanoma.

The term "prevent" in the context of tumour metastasis means that a tumour is precluded from metastasing in the presence of the inhibitor of the invention. On the molecular level the prevention is **characterized in that** the recruitment of platelets by tumour cells does not result in the decrease of endothelial barrier function to the extent that tumour cell extravasation occurs. While the term "prevent" thus encompasses abolition of metastasis, i.e. no metastasis occurs, it also relates to a decrease in the risk and/or rate of metastasis of a tumour occurring, e.g. by at least (for each value) 30%, 40%, or 50% such as at least (for each value) 55%, 60%, 65%, 70% or 75%, preferred by at least (for each value) 80% or 85%, more preferred at least (for each value) 90% or 95% and most preferred by 100%, i.e. abolition of tumour metastasis, as mentioned before. In other words, the term "prevent" also encompasses the meaning of reducing the risk and/ or rate of tumour metastasis. As will be understood by the person skilled in the art, even a reduction in the risk and/or rate of metastasis occurring will be beneficial as it may render the disease more manageable from a clinical standpoint and may increase the life expectancy of the patient afflicted with a tumour, in particular with regard to tumours characterized by a high rate and/or risk to metastasize. It is also understood that the inhibitors as defined herein can be used for the prevention of metastasis of a tumour in subjects afflicted with a tumour independent from the level of expression or activity of the P2Y2 receptor in the tumour tissue. In other words, if the P2Y2 receptor activity or expression is elevated in tumour tissue in contrast to control levels (derived from the same tissue kind but not afflicted with a tumour from the same subject or other subjects) metastasis of the tumour can be prevented using the inhibitors as defined herein. Similarly, tumour metastasis can be prevented in subjects afflicted with a tumour having an elevated P2Y2 receptor activity or expression in contrast to control levels.

It is understood in accordance with the invention that the inhibitors as defined herein can be used for the prevention of metastasis on tumours that have not yet begun to metastasize or that already metastasize.

The term "lead compound" as used herein refers to a compound that can be classified as an inhibitor of the P2Y2 receptor or the P2Y2 receptor signaling pathway as defined herein above. Such lead compounds are used as starting compounds for developing drugs to prevent the metastasis of tumours in that they may be optimized with regard to, e.g., their potency, their pharmacokinetic profile or their suitability to be used in a certain pharmaceutical formulation, so as to arrive at a compound which may be, advantageously, safely and effectively used in a pharmaceutical composition. Methods and tools for the optimization of the pharmacological properties of compounds identified in screens, the lead compounds, are known in the art. For example, in silico tools for optimizing lead compounds are known in the art and described, e.g., in Cruciani et al., European Journal of Pharmaceutical Sciences, vol. 11, suppl. 2, p. S29-S39 (2000). Furthermore, high-throughput approaches for evaluating properties of lead compounds have been described in Tarbit and Berman, Current Opinion in Chemical Biology, vol. 2, issue 3, p. 411-416 (1998).

In a more preferred embodiment of the methods of the invention, the optimisation comprises modifying the inhibitor of the P2Y2 receptor or the inhibitor of the P2Y2 receptor signaling pathway to achieve: i) modified spectrum of activity, organ specificity, and/or ii) improved potency, and/or iii) decreased toxicity (improved therapeutic index), and/or iv) decreased side effects, and/or v) modified onset of therapeutic action, duration of effect, and/or vi) modified pharmacokinetic parameters (resorption, distribution, metabolism and excretion), and/or vii) modified physico-chemical parameters (solubility, hygroscopicity, color, taste, odor, stability, state), and/or viii) improved general specificity, organ/tissue specificity, and/or ix) optimised application form and route by (a) esterification of carboxyl groups, or (b) esterification of hydroxyl groups with carboxylic acids, or (c) esterification of hydroxyl groups to, e.g. phosphates, pyrophosphates or sulfates or hemi-succinates, or (d) formation of pharmaceutically acceptable salts, or (e) formation of pharmaceutically acceptable complexes, or (f) synthesis of pharmacologically active polymers, or (g) introduction of hydrophilic moieties, or (h) introduction/exchange of substituents on aromates or side chains, change of substituent pattern, or (i) modification by introduction of isosteric or bioisosteric moieties, or (j) synthesis of homologous compounds, or (k) introduction of branched side chains, or (1) conversion of alkyl substituents to cyclic analogues, or (m) derivatisation of hydroxyl groups to ketales, acetales, or (n) N-acetylation to amides, phenylcarbamates, or (o) synthesis of Mannich bases, imines, or (p) transformation of ketones or aldehydes to Schiff's bases, oximes, acetales, ketales, enolesters, oxazolidines, thiazolidines or combinations thereof.

The various steps recited above are generally known in the art, as described above. They include or rely on quantitative structure-activity relationship (QSAR) analyses (Kubinyi (1992) "Hausch-Analysis and Related Approaches", VCH Verlag, Weinheim), combinatorial biochemistry, classical chemistry and others (see, for example, Holzgrabe and Bechtold (2000) Deutsche Apotheker Zeitung 140(8), 813).

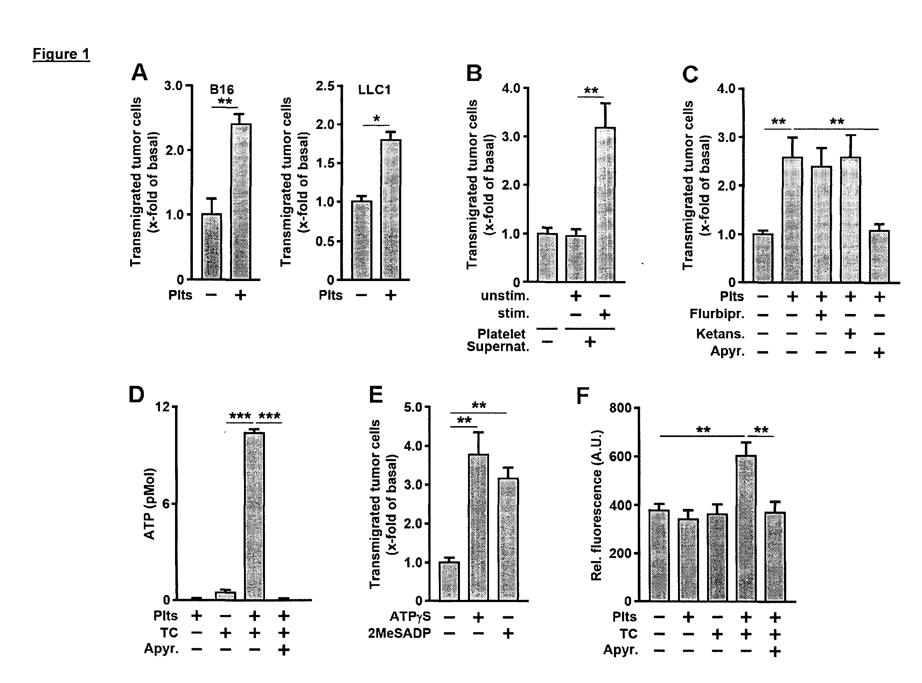

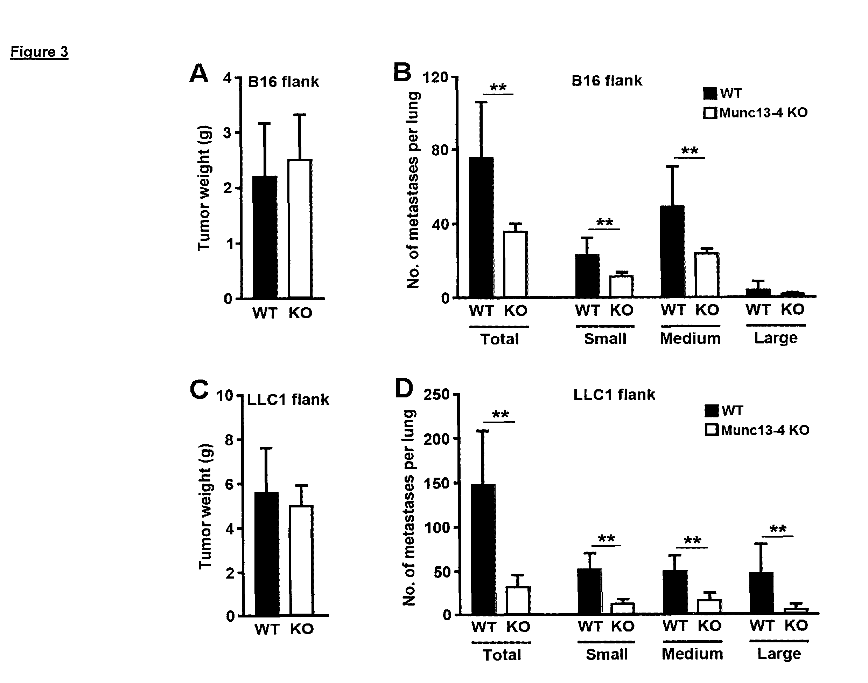

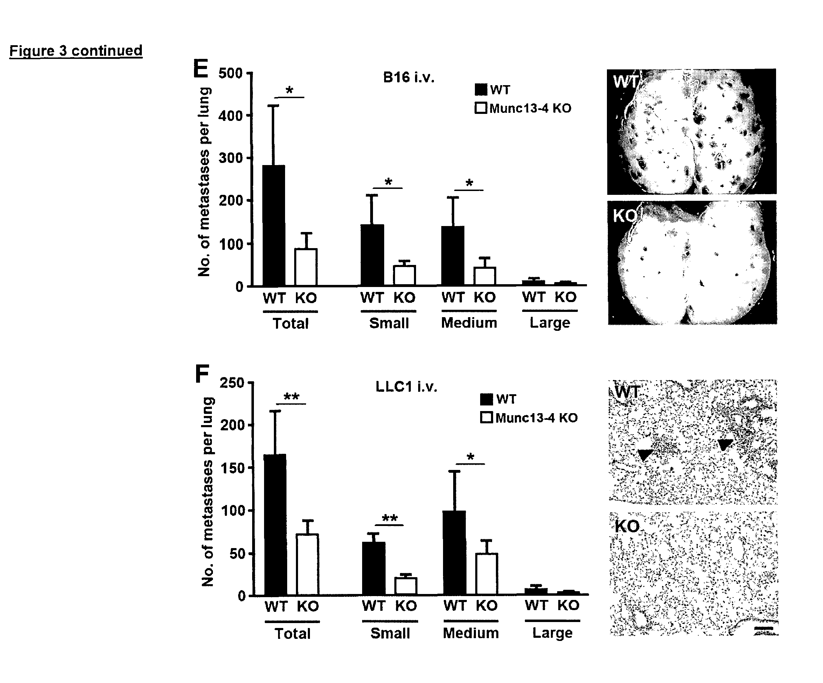

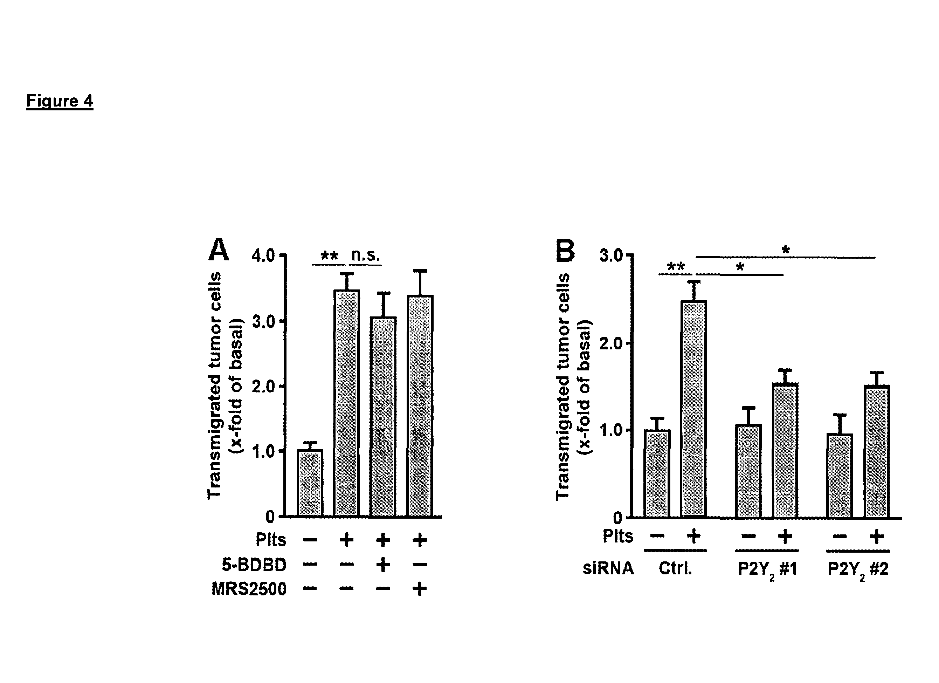

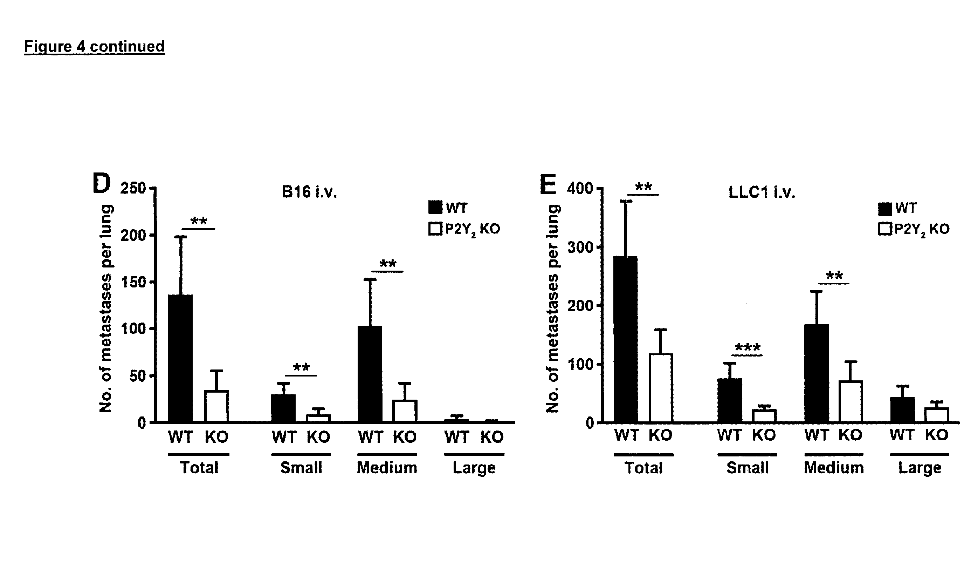

It was surprisingly found that the P2Y2 receptor on endothelial cells is involved in the platelet-mediated tumour cell extravasation and that inhibition of the P2Y2 receptors *in vitro* significantly reduced platelet-mediated tumour cell transmigration (see Fig. 4B). A similar result was achieved in mice lacking P2Y2 receptors injected with tumour cells in contrast to wild type mice (Fig. 4D and E). Furthermore, it could be shown that degradation of P2Y2's ligand ATP and ADP ablated platelet-dependent facilitation of tumour cell transmigration (see Fig. 1C). This evidences that adenine nucleotides released from dense granules of tumour cell-activated platelets promote an opening of the endothelial barrier and a transmigration of tumour cells through the endothelial layer. Corroborating the latter, Munc13-4 deficient platelets lost the ability to enhance transmigration of tumour cells (see Fig 2G), as Munc13-4 deficiency leads to the inability of said platelets to release ATP stored in platelet dense granules in response to tumour cells (see Figs. 2E and F). This finding was confirmed *in vivo* in mice (see Fig. 3). These findings establish the P2Y2 receptor and the P2Y2 receptor signaling pathway as a pharmaceutical target whose inhibition leads to the prevention tumour cell metastasis as outlined herein. Prior to the invention, the P2Y2 receptor was hypothesized to be potentially involved in various diseases, e.g., cancer, by way of abnormally low or high activity or expression of P2Y2 in the diseased tissue in comparison to healthy tissue (WO2005/100990) Thus, it was speculated that treatment of said diseases can be achieved by modulating said abnormal activity or expression in said diseased tissue. As P2Y2 expression was shown to be not uniformly up- or downregulated in various different cancer tissues which are capable of metastasizing, some cancers would be treated by up- or downregulating the expression or activity of P2Y2 in the diseased cells. In view of the teaching of the present invention, treating a cancer by upregulating its P2Y2 expression or activity would boost the capacity of the cancer to metastasize. The new findings described herein, provide a new rationale that is not dependent on abnormally high or low P2Y2 expression or activity within the diseased tissue, but targets tissue not affected by a tumour and displaying normal activity and expression of the P2Y2 receptor and is independent from the expression or activity of the P2Y2 receptor in the tumour tissue. As such, subjects may be administered the inhibitors as defined herein to prevent metastasis of tumours that would based on the prior art teaching be considered ineligible for treatment with pharmaceutically active agents leading to the decrease of the activity or expression of the P2Y2 receptor.

In a preferred embodiment, the inhibitor is an antibody or a fragment or derivative thereof, a siRNA, a shRNA, a miRNA, a ribozyme, an aptamer, an antisense nucleic acid molecule, a small molecule or a modified version of these inhibitors.

The term "antibody" as used in accordance with the present invention comprises, for example, polyclonal or monoclonal antibodies. Furthermore, also derivatives or fragments thereof, which still retain the binding specificity, are comprised in the term "antibody". Antibody fragments or derivatives comprise, inter alia, Fab or Fab' fragments as well as Fd, F(ab')₂, Fv or scFv fragments; see, for example Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also includes embodiments such as chimeric (human constant domain, non-human variable domain), single chain and humanized (human antibody with the exception of non-human CDRs) antibodies.

Various techniques for the production of antibodies are well known in the art and described, e.g. in Harlow and Lane (1988) and (1999), loc. cit. Thus, the antibodies can be produced by peptidomimetics, Further, techniques described for the production of single chain antibodies (see, inter alia, US Patent 4,946,778) can be adapted to produce single chain antibodies specific for the target of this invention. Also, transgenic animals or plants (see, e.g., US patent 6,080,560) may be used to express (humanized) antibodies specific for the target of this invention. Most preferably, the antibody is a monoclonal antibody, such as a human or humanized antibody. For the preparation of monoclonal antibodies, any technique which provides antibodies produced by continuous cell line cultures can be used. Examples for such techniques are described, e.g. in Harlow and Lane (1988) and (1999), loc. cit. and include the hybridoma technique (as originally demonstrated by Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BlAcore system can be used to increase the efficiency of phage antibodies which bind to an epitope of the P2Y2 receptor or a target molecule within the P2Y2 receptor signaling pathway (Schier, Human Antibodies Hybridomas 7 (1996), 97-105; Malmborg, J. lmmunol. Methods 183 (1995),7-13). It is also envisaged in the context of this invention that the term "antibody" comprises antibody constructs which may be expressed in cells, e.g. antibody constructs which may be transfected and/or transduced via, inter alia, viruses or plasmid vectors.

Aptamers are nucleic acid molecules or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

More specifically, aptamers can be classified as nucleic acid aptamers, such as DNA or RNA aptamers, or peptide aptamers. Whereas the former normally consist of (usually short) strands of oligonucleotides, the latter preferably consist of a short variable peptide domain, attached at both ends to a protein scaffold.

Nucleic acid aptamers are nucleic acid species that, as a rule, have been engineered through repeated rounds of in vitro selection or equivalently, SELEX (systematic evolution of ligands by exponential enrichment) to bind to various molecular targets such as small molecules, proteins, nucleic acids, and even cells, tissues and organisms.

Peptide aptamers usually are peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A is the most commonly used scaffold protein, the variable peptide loop being inserted within the redox-active site, which is a -Cys-Gly-Pro-Cys-loop in the wild protein, the two cysteins lateral chains being able to form a disulfide bridge. Peptide aptamer selection can be made using different systems, but the most widely used is currently the yeast two-hybrid system.

Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as in vivo diagnostic imaging. Several modifications, such as 2'-fluorine-substituted pyrimidines, polyethylene glycol (PEG) linkage, fusion to albumin or other half life extending proteins etc. are available to scientists such that the half-life of aptamers can be increased for several days or even weeks.

The term "peptide" as used herein describes a group of molecules consisting of up to 30 amino acids, whereas the term "polypeptide" as used herein describes a group of molecules consisting of more than 30 amino acids. The group of peptides and polypeptides are referred to together with the term "(poly)peptide". Also encompassed by the term "(poly)peptide" are proteins as well as fragments of proteins of more than 30 amino acids. The term "fragment of protein" in accordance with the present invention refers to a portion of a protein comprising at least the amino acid residues necessary to maintain the biological activity of the protein. Preferably, the amino acid chains are linear. (Poly)peptides may further form multimers consisting of at least two identical or different molecules. The corresponding higher order structures of such multimers are correspondingly termed homo- or heterodimers, homo- or heterotrimers etc. Furthermore, peptidomimetics of such (poly)peptides where amino acid(s) and/or peptide bond(s) have been replaced by functional analogues are also encompassed by the invention. Such functional analogues include all known amino acids other than the 20 gene-encoded amino acids, such as selenocysteine. The term "(poly)peptide" also refers to naturally modified (poly)peptides where the modification is effected e.g. by glycosylation, acetylation, phosphorylation and similar modifications which are well known in the art.

It is also well known that (poly)peptides are not always entirely linear. For instance, (poly)peptides may be branched as a result of ubiquitination, and they may be circular, with or without branching, generally as a result of post-translation events, including natural processing event and events brought about by human manipulation which do not occur naturally. Circular, branched and branched circular (poly)peptides may be synthesized by non-translational natural processes and by synthetic methods. The modifications can be a function of how the (poly)peptide is made. For recombinant (poly)peptides, for example, the modifications will be determined by the host cells posttranslational modification capacity and the modification signals in the amino acid sequence. Accordingly, when glycosylation is desired, a (poly)peptide should be expressed in a glycosylating host, generally an eukaryotic cell, for example Cos7, HELA or others. The same type of modification may be present in the same or varying degree at several sites in a given (poly)peptide. Also, a given (poly)peptide may contain more than one type of modification.

In accordance with the present invention, the term "small interfering RNA (siRNA)", also known as short interfering RNA or silencing RNA, refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

siRNAs naturally found in nature have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene of which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1-5 nucleotides, more preferably from 1-3 nucleotides and most preferably 2 nucleotides. The other end may be bluntended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have a 2-nt 3'-overhang. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant. Delivery of siRNA may be accomplished using any of the methods known in the art, for example by combining the siRNA with saline and administering the combination intravenously or intranasally or by formulating siRNA in glucose (such as for example 5% glucose) or cationic lipids and polymers can be used for siRNA delivery in vivo through systemic routes either intravenously (IV) or intraperitoneally (IP) (Fougerolles et al. (2008), Current Opinion in Pharmacology, 8:280-285; Lu et al. (2008), Methods in Molecular Biology, vol. 437: Drug Delivery System - Chapter 3: Delivering Small Interfering RNA for Novel Therapeutics). The activity and specificity of siRNAs can be altered by various modifications such as, e.g., by inclusion of a blocking group at the 3' and 5' ends, wherein the term "blocking group refers to substituents of that can be attached to oligonucleotides or nucleomonomers, either as protecting groups or coupling groups for synthesis (cf. WO 98/13526, EP 2221377 B1), by inclusion of agents that enhance the affinity to the target sequence such as intercalating agents (e.g., acridine, chlorambucil, phenazinium, benzophenanthirdine), attaching a conjugating or complexing agent or encapsulating it to facilitate cellular uptake, or attaching targeting moieties for targeted delivery.

A short hairpin RNA (shRNA) is a sequence of RNA that makes a tight hairpin turn that can be used to silence gene expression via RNA interference. shRNA uses a vector introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it. si/shRNAs to be used in the present invention are preferably chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAI.

Further molecules effecting RNAi include, for example, microRNAs (miRNA). Said RNA species are single-stranded RNA molecules which, as endogenous RNA molecules, regulate gene expression. Binding to a complementary mRNA transcript triggers the degradation of said mRNA transcript through a process similar to RNA interference. Accordingly, miRNA may be employed as an inhibitor of the P2Y2 receptor or an inhibitor of the P2Y2 receptor signaling pathway.

A ribozyme (from ribonucleic acid enzyme, also called RNA enzyme or catalytic RNA) is an RNA molecule that catalyzes a chemical reaction. Many natural ribozymes catalyze either their own cleavage or the cleavage of other RNAs, but they have also been found to catalyze the aminotransferase activity of the ribosome. Non-limiting examples of well-characterized small self-cleaving RNAs are the hammerhead, hairpin, hepatitis delta virus, and in vitro-selected lead-dependent ribozymes, whereas the group I intron is an example for larger ribozymes. The principle of catalytic self-cleavage has become well established in the last 10 years. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: An interesting region of the RNA, which contains the GUC (or CUC) triplet, is selected. Two oligonucleotide strands, each usually with 6 to 8 nucleotides, are taken and the catalytic hammerhead sequence is inserted between them. Molecules of this type were synthesized for numerous target sequences. They showed catalytic activity in vitro and in some cases also in vivo. The best results are usually obtained with short ribozymes and target sequences.

A recent development, also useful in accordance with the present invention, is the combination of an aptamer recognizing a small compound with a hammerhead ribozyme. The conformational change induced in the aptamer upon binding the target molecule is supposed to regulate the catalytic function of the ribozyme.

The term "antisense nucleic acid molecule" is known in the art and refers to a nucleic acid which is complementary to a target nucleic acid. An antisense molecule in accordance with the invention is capable of interacting with the target nucleic acid, more specifically it is capable of hybridizing with the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

A "small molecule" as used herein may be, for example, an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 amu, or less than about 1000 amu such as less than about 500 amu, and even more preferably less than about 250 amu. The size of a small molecule can be determined by methods well-known in the art, e.g., mass spectrometry. The small molecules may be designed, for example, based on the crystal structure of the target molecule, where sites presumably responsible for the biological activity, can be identified and verified in in vivo assays such as in vivo high-throughput screening (HTS) assays.

The term "modified versions of these inhibitors" in accordance with the present invention refers to versions of the inhibitors that are modified to achieve i) modified spectrum of activity, organ specificity, and/or ii) improved potency, and/or iii) decreased toxicity (improved therapeutic index), and/or iv) decreased side effects, and/or v) modified onset of therapeutic action, duration of effect, and/or vi) modified pharmacokinetic parameters (resorption, distribution, metabolism and excretion), and/or vii) modified physico-chemical parameters (solubility, hygroscopicity, color, taste, odor, stability, state), and/or viii) improved general specificity, organ/tissue specificity, and/or ix) optimised application form and route. This can be achieved by performing the methods as described herein above in relation to lead compounds.

In another preferred embodiment of the invention, the inhibitor of the P2Y2 receptor signaling pathway is an inhibitor of G-proteins G_{q}/G₁₁, phospholipase Cβ and/or protein kinase C (PKC).

As used in accordance with the preset invention, an "inhibitor of G-proteins G_{q}/G₁₁" is meant to relate to an inhibitor that inhibits the biological activity or function of said G-proteins being the binding to the P2Y2 receptor, the activation of phospholipase Cβ by G_{q}/G₁₁ and/or the activation of G_{q}/G₁₁ by GTP. G_{q}/G₁₁ couple P2Y2 receptors to intracellular signalling pathways, in particular β-isoforms of phospholipase C. The α-subunits of G_{q}/G₁₁. Gα_{q} and Gα₁₁, could be targeted by inhibitors as defined herein resulting in reduced expression or increased degradation of the protein. Function of G_{q}/G₁₁ may be affected by blocking the interaction between the receptor and the G-protein, by blocking the binding of GTP and/or GDP to the G-protein, by increasing the inactivation of the G-proteins through increased GTPase activity using compounds which stimulate the intrinsic GTPase activity of the α-subunit of Gq and/or G₁₁, or by delivering GTPase activating proteins of the "regulator of G-protein signalling" (RGS)-family. Also, interaction between the activated G-protein and downstream effectors (e.g. β-isoforms of phospholipase C) may be inhibited. In other words, said inhibition may be achievable, e.g., by an inhibitor that reduces the amount of the G-proteins G_{q}/G₁₁ available for binding to the P2Y2 receptor. Such an inhibitor may act on G_{q}/G₁₁ directly, such as for example by reducing its expression levels or its binding abilities to the P2Y2 receptor or it may reduce the level of GTP bound by the G-protein thereby preventing G_{q}/G₁₁ activation. Alternatively, an inhibitor may compete with said G-proteins for binding to the P2Y2 receptor, it may block the G-protein binding site binding site on the P2Y2 receptor or the P2Y2 receptor binding site on said G-proteins. For example, the inhibitor YM-254890 can be used. YM-254890 inhibits the guanine nucleotide exchange on Gα_{q}/Gα₁₁ and thereby blocks the activation step of the G-protein (N. Mizuni, H. Itoh, Neurosignals 17, 42-54 (2009)). Structural information on YM-254890 can be found in J. Takasaki et al., J. Biol. Chem. 279, 47438-47445 (2004); A. Nishimura et al., Proc. Natl. Acad. Sci. U. S. A. 107, 13666-13671 (2010)). The substance is, e.g., produced by Astellas Pharma Inc. 21 Miyukigaoka, Tsukuba, Ibaraki 305-8585, Japan; Monash University in Melbourne, Australia, has reported the total synthesis of the highly selective Gα_{q}/Gα₁₁ protein inhibitor YM-254890 (Monash University Pharmacy and Pharmaceutical Sciences Department, http://www.pharm.monash.edu.au/courses/honours/mipsprojects/ddbiol/ddb-11-project.html)

An inhibitor of "phospholipase Cβ" is meant to relate to an inhibitor that inhibits the biological activity or function of said phospholipase Cβ (PLCβ). The phospholipase C β-isoforms are regulated through P2Y2 and G_{q}/G₁₁. PLCβ isoforms catalyze the formation of inositol-1,4,5-triphosphate and diacylglycerol from phosphatidylinositol-4,5-bisphosphate. Inhibitors may interfere with the interaction of G-protein α-subunits or G-protein βγ-subunits with phospholipase C β-isoforms or may block the access of the substrate (phosphatidylinositol-4,5-bisphosphate) to the enzyme, or they may block the conversion of the substrate into the products. The exemplary mentioned methods of action of inhibitors described above for an inhibitor of the G-proteins G_{q}/G₁₁ also applies *mutatis mutandis* and in other parts of the specification to an inhibitor suitable for inhibiting phospholipase Cβ. An exemplary inhibitor is U73122. Structural and further information on U73122 can be found in the following three references: J. E. Bleasdale et al., J. Pharmacol. Exp. Ther. 255, 756-768 (1990); R. J. Smith et al., J. Pharmacol. Exp. Ther. 278, 320-329 (1996); T. J. Shi et al., Proc. Natl. Acad. Sci. U. S. A. 105, 20004-20008 (2008).

An inhibitor of "protein kinase C (PKC)" is meant to relate to an inhibitor that inhibits the biological activity or function of said PKC. Protein kinase C (PKC) is a family of protein kinases of which several isoforms can be activated by diacylglycerol, a product of phospholipase Cβ enzyme. Thus PKC is a component of signalling pathways downstream of P2Y2. There are multiple inhibitors of PKC available (as described, e.g., in G. X. Shen, Curr. Drug Targets Cardiovasc. Haematol. Disord. 3, 301-307 (2003)). The exemplary mentioned methods of action of inhibitors described above for an inhibitor of the G-proteins G_{q}/G₁₁ also applies *mutatis mutandis* and in other parts of the specification to an inhibitor suitable for inhibiting PKC. Exemplary inhibitors include isoquinoline sulphonamides, HA1004, staurosporine, calphostin C, UCN-01, Ro317208, Ro3l8220, GF109203, Go6983, LY333531, rottlerin or 379196 as described and further detailed in G. X. Shen, Curr. Drug Targets Cardiovasc. Haematol. Disord. 3, 301-307 (2003).

As evident from the information given herein above and in the example section, the P2Y2 receptor plays a crucial role in the transmigration of tumour cells via the endothelial barrier. Thus, it is expected that the inhibition of factors immediately downstream of the P2Y2 receptor will be beneficial to achieve effects similar to those achievable by inhibition of the P2Y2 receptor itself.

As mentioned herein above, also molecules upstream from the P2Y2 receptor that are part of the P2Y2 receptor signaling pathway as defined herein provide suitable pharmaceutical targets whose inhibition leads to the prevention of tumour cell metastasis. For example, ATP and ADP as targets can be degraded by using, e.g., apyrase as inhibitor in accordance with the invention. Also, and in line with the above definitions (which apply *mutatis mutandis* also to this embodiment) for inhibitors, an inhibitor may block the binding site for ATP or ADP on the P2Y2 receptor or vice versa.

Another, preferred target is the inhibition of Munc13-4 to prevent release of ATP from the dense granules of platelets. This can be achieved by inhibiting the biological function or activity of Munc13-4. As outlined herein above, Munc13-4 is not involved in P2Y2 signalling but is required for the release of ATP and ADP from platelets which then serve as agonists of P2Y2. A suitable inhibitor may interfere with the formation, intracellular transport or degradation of the Munc13-4 protein. Alternatively, an inhibitor may block the interaction between Munc13-4 and other components of the exocytotic machinery, including SNARE proteins or the protein Munc-18. The exemplary mentioned methods of action of inhibitors described above for an inhibitor of the G-proteins G_{q}/G₁₁ also applies *mutatis mutandis* and in other parts of the specification to an inhibitor suitable for inhibiting Munc13-4.

In a preferred embodiment, the inhibitor of the P2Y2 receptor is a siRNA with the nucleic acid sequence of any one of SEQ ID NOs: 1 to 8.

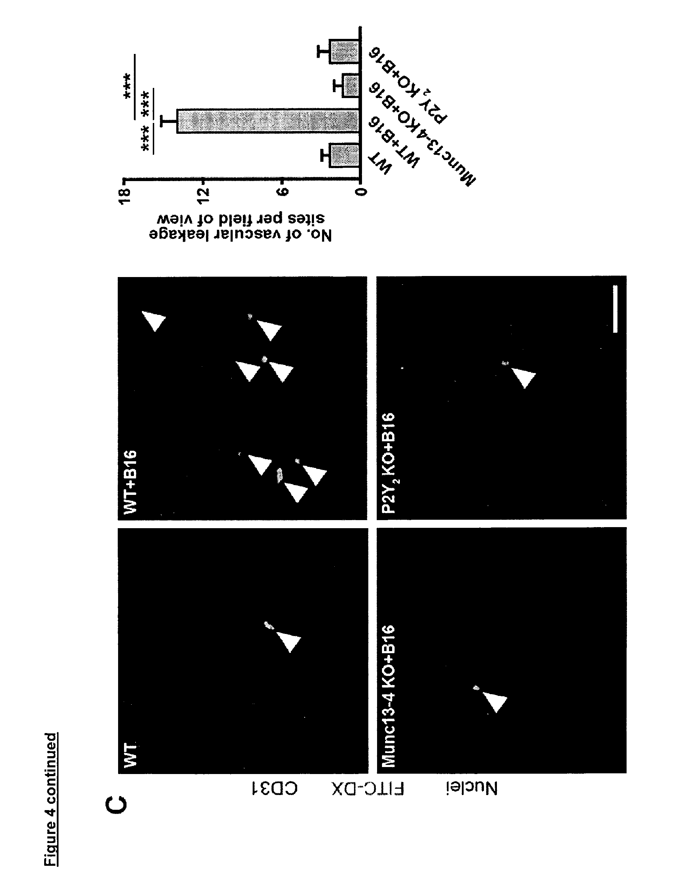

As demonstrated in the example section (see also Fig. 4), a suitable inhibitor of P2Y2 is siRNA. Without limitation, exemplary inhibitors for the human P2Y2 receptor are siRNAs having the nucleic acid sequence of any one of SEQ ID NOs: 5 to 8, whereas siRNA inhibiting the murine P2Y2 receptor have the nucleic acid sequence of any one of SEQ ID NOs: 1 to 4.

Also preferred as inhibitors of the P2Y2 receptor are 4-Phenylamino-substituted 1-amino-2-sulfoanthraquinones as described in detail in S. Weyler et al., Bioorg. Med. Chem. Lett. 18, 223-227 (2007), in particular those listed in Table 2 therein. A particularly preferred inhibitor 1-amino-4-(2-methoxyphenyl)-2-sulfoanthraquinone (also described in S. Weyler et al., Bioorg. Med. Chem. Lett. 18, 223-227 (2007)).

In a further preferred embodiment, the inhibitor has admixed thereto or is associated in a separate container with a further pharmaceutically active agent.

The skilled person is well-aware of the meaning of the term "pharmaceutically active agent" in that it relates to an agent that elicits an effect in a subject that renders it useful in the treatment of a disease either alone or in combination with further active agents. In the present case an inhibitor alone or as part of a pharmaceutical composition may be combined as an admixture with any other pharmaceutically active agent. Envisaged are, for example, combinations of anti-tumour agents with the inhibitors according to the invention. Anti-tumour agents are used in chemotherapy, are known in the art and include, e.g., antineoplastic drugs that kill tumour cells or inhibit their proliferation. Anti-tumour agents can be classified by their method of action or origin and include, for example, alkylating agents, anti-metabolites, plant alkaloids and terpenoids, topoisomerase inhibitors and cytotoxic antibodies. Exemplary alkylating agents include, e.g., nitrogen mustards (mechlorethamine, cyclophosphamide, ifosfamide, melphalan, chlorambucil), methylhydrazine derivative (Procarbazine (N-methylhydrazine, MIH)), alkyl sulfonate (busulfan), nitrosoureas (carmustine (BCNU), streptozocin (streptozotocin), bendamustine), triazenes (dacarbazine (DTIC, dimethyltriazenoimidazole carboxamide), temozolomide) platinum coordination complexes (cisplatin, carboplatin, oxaliplatin). Exemplary antimetabolites include, e.g., folic acid analogs (methotrexate (amethopterin), pemetrexed), pyrimidine analogs (fluorouracil (5-fluorouracil; 5-FU), capecitabine, cytarabine (cytosine arabinoside), gemcitabine, 5-aza-cytidine, deoxy-5-aza-cytidine), purine analogs and related inhibitors (mercaptopurine (6-mercaptopurine; 6-MP), pentostatin (2'-deoxycoformycin), fludarabine, clofarabine, nelarabine). Exemplary natural products (e.g., plant alkaloids and terpenoids) include, e.g., vinca alkaloids (vinoblastine, vinorelbine, vincristine), taxanes (paclitaxel, docetaxel), epipodophyllotoxins (etoposide, teniposide), camptothecins (topotecan, irinotecan), antibiotics (dactinomycin (actinomycin D), daunorubicin (daunomycin, rubidomycin), doxorubicin), echinocandins (yondelis), anthracenedione (mitoxantrone, bleomycin, mitomycin C), enzymes (L-asparaginase). Exemplary hormones and antagonists include, e.g., adrenocortical suppressants (mitotane (o.p'-DDD)), adrenocortico-steroids (prednisone (other equivalent preparations available)), progestins (hydroxyprogesterone caproate, medroxyprogesterone acetate, megestol acetate), estrogens (diethylstilbestrol, ethinyl, estradiol (other preparations available), anti-Estrogens (tamoxifen, toremifene), aromatase inhibitors (anastrozole, letrozole, exemestane), androgens (testosterone propionate, fluoxymesterone (other preparations available)), anti-androgen (flutamide, casodex), GnRH analog (leuprolide). Exemplary further anti-tumour agents include, e.g., substituted urea (hydroxyurea), differentiating agents (tretinoin, arsenic trioxide, histone deacetylase, inhibitor (vorinostat)), protein tyrosine kinase inhibitors (imatinib, dasatinib, nilotinib, gefitinib, erlotinib, sorafenib, sunitinib, lapatinib), proteasome inhibitor (bortezomib), biological response modifiers (interferon-alfa, interleukin-2), immunomodulators (thalidomide, lenalidomide), mTOR inhibitors (temsirolimus, everolimus), monoclonal antibodies.

Also envisaged is the combination of inhibitors according to the invention with other metastasis preventing agents. Examples of such combinations can be found in G. Y. Perret, M. Crépin, Fundam. Clin. Pharmacol. 22, 465-492 (2008). The skilled person is in the position to identify a suitable treatment regimen of the combination of active agent and inhibitor as defined herein in the case of the latter being admixed or the further pharmaceutically active agent being provided separately in a container by routine methods. A container may take the form of, e.g., a vial. The vial may, in addition to the pharmaceutically active agent, comprise preservatives or buffers for storage, media for maintenance and storage. The pharmaceutically active agent may further be comprised in a pharmaceutical composition. The definitions given herein above with regard to the term "pharmaceutical composition" apply *mutatis mutandis* also to this embodiment.

It is appreciated by the skilled person that this embodiment is suitable to provide a more complete clinical approach in disease management of subjects afflicted with tumours as defined herein above, i.e. known to metastasize or speculated to metastasize.

The explanations, preferred embodiments, examples and definitions given herein above also apply *mutatis mutandis* to the method of the invention as detailed below.

In another embodiment the invention relates to a method of preventing the metastasis of tumours comprising administering a pharmaceutically effective amount of an inhibitor of the P2Y2 receptor or an inhibitor of the P2Y2 receptor signaling pathway to a subject in need thereof.

In a preferred embodiment of the method of the invention, the inhibitor is an antibody or a fragment or derivative thereof, a siRNA, a shRNA, a miRNA, a ribozyme, an aptamer, an antisense nucleic acid molecule, a small molecule or a modified version of these inhibitors.

In another preferred embodiment of the method of the invention, the inhibitor of the P2Y2 receptor signaling pathway is an inhibitor of G-proteins G_{q}/G₁₁, phospholipase Cβ and/or protein kinase C (PKC).

In a further preferred embodiment of the method of the invention, the inhibitor of the P2Y2 receptor is a siRNA with the nucleic acid sequence of any one of SEQ ID NO: 1 to 8.

In a preferred embodiment of the invention, the method further comprises administering a further pharmaceutically active agent.

The figures show:

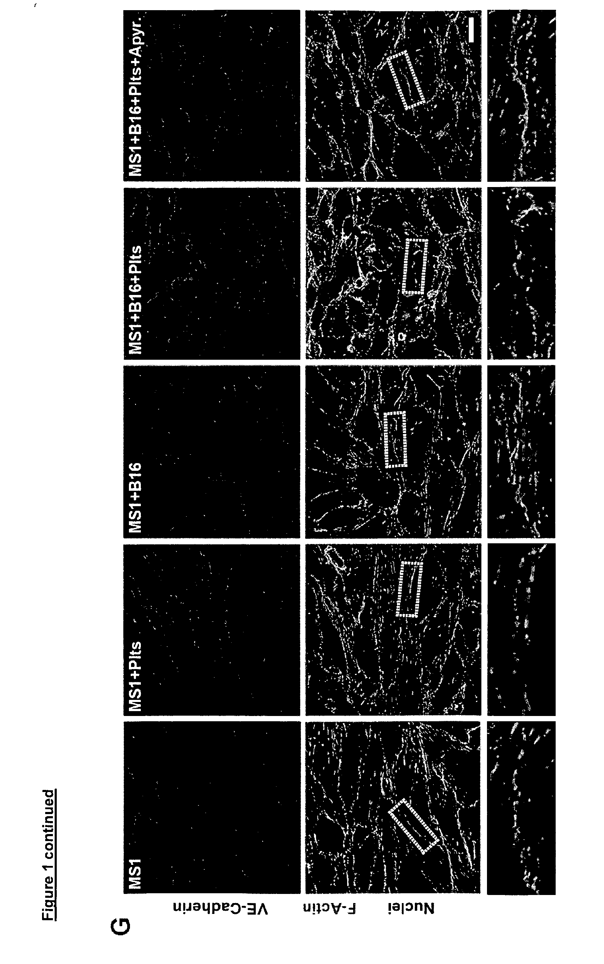

**Figure 1****:** Platelets stimulate tumour cell transmigration *in vitro* through release of nucleotides. **(A,B)** The indicated murine tumour cells were seeded on murine microvascular endothelial cells (MS1), and tumour cell transmigration was determined in the absence or presence of platelets (-/+ Plts) (A) or supernatants (supernat.) of platelets preincubated in the absence (unstim.) or presence of tumour cells (stim.) **(B)** (n=5). **(C)** Effect of the COX-inhibitor flurbiprofen (Flurbipr., 100 nM), the 5-HT₂ receptor antagonist ketanserin (Ketans., 3 nM) or the ATP/ADP degrading enzyme apyrase (Apyr., 20 U/ml) on platelet (Plts)-dependent stimulation of B16 cell transendothelial migration (n=5). **(D)** Release of ATP from platelets (Plts) alone, B16 cells (TC) alone or a mixture of tumour cells and platelets (1:100) in the absence or presence of apyrase (Apyr., 20 U/ml). **(E)** Effect of the stable ATP and ADP analogues ATPγS (10 µM) and 2MeSADP (10 µM) on transendothelial migration of B16 cells (n=5). **(F)** Effect of B16 cells (TC) and platelets (Pits) alone or together in the absence and presence of apyrase (Apyr., 20 U/ml) on the permeability of MS1 endothelial cell layer as determined by permeability for FITC-dextran (n=5). **(G)** Morphology of MS1 endothelial cells exposed to supernatants from B16 cells alone or B16 cells incubated with platelets (Pits) in the absence and presence of apyrase (Apyr.). Cells were stained for VE-cadherin (red), F-actin (green) and cell nuclei (blue). Boxes indicate enlarged areas at the bottom. Bar length: 5 µm. Shown are mean values ± S.E.M. (A-C,E,F) or S.D. (D); *, *P*<0.05; **, *P*<0.01; ***, *P*<0.001; n.s., not significant.
**Figure 2****:** Loss of platelet dense granule secretion blocks platelet-mediated tumour cell transmigration *in vitro.* **(A-F)** Effect of thrombin (A,C,E) or tumour cells (B,D,F) on release of platelet factor 4 (PF4) (A,B), von Willebrand factor (vWF) (C,D) or ATP (E,F) from wild-type (WT) or Munc13-4-deficient platelets (KO) or in the absence of platelets (-Plts) (n=3-5). **(G)** Effect of wild-type (WT) and Munc13-4-deficient platelets (KO) on transendothelial migration of B16 (left panel) and LLC1 tumour cells (right panel) (n=5). Shown are mean values ± S.D. (A-F) or S.E.M. (G); *, *P*<0.05; **, *P*<0.01; ***, *P*<0.001; n.s., not significant.
**Figure 3****:** Reduced tumour cell metastasis in mice with defective dense granule secretion. **(A-D)** B16 (A,B) or LLC1 cells (C,D) were injected subcutaneously into the flank of wild-type (WT) or Munc13-4-deficient mice (KO). After 21 days, animals were sacrificed, primary tumour weight was determined (A,C), and lungs of animals were histologically analyzed for the occurrence of metastases. Total number as well as the number of small (diameter: <0.1 mm), medium-sized (diameter: 0.1-1.0 mm) or large (diameter: >1.0 mm) lung metastases was determined (B,D) (n=7-10). **(E,F)** B16 (E) or LLC1 cells (F) were injected i.v. into wild-type (WT) or Munc13-4-deficient mice (KO), and 12 days later, lung metastases were evaluated as in B and D (n=6-10). The images show representative lung surface metastases (E) or stained lung sections (F) from wild-type (WT) or Munc13-4-deficient mice (KO). Bar length: 100 µm. Arrowheads indicate metastases. Shown are mean values ± S.D.; *, *P*<0.05; **, P<0.01; n.s., not significant.
**Figure 4****:** Platelet-mediated facilitation of tumour cell extravasation and metastasis involves P2Y₂ receptors. **(A)** Effect of the P2X₄ receptor antagonist 5-BDBD (1 µM) and of the P2Y₁ antagonist MRS2500 (100 nM) on platelet-stimulated transendothelial migration of B16 cells (n=5). **(B)** Endothelial cells were transfected with control siRNA or with two different siRNAs directed against the mRNA encoding P2Y₂. Thereafter, transmigration of B16 cells was determined in the absence or presence of platelets (-/+Plts) (n=5). **(C)** FITC-dextran extravasation in the lungs of wild-type (WT), Munc13-4-deficient (Munc13-4 KO) or P2Y₂-deficient (P2Y₂ KO) mice 3 hours after i.v. injection of buffer or B16 cells. Shown are representative images (3x magnification from original) of lung sections stained for CD31 (red) or cell nuclei (blue). Extravasation sites of FITC-dextran (FITCDX; green) are indicated by arrowheads. The bar diagram shows a quantification of extraluminal FITC-positive sites (n=4-8). Bar length: 20 µm. **(D,E)** B16 (D) and LLC1 cells (E) were injected i.v. in wild-type (WT) and P2Y₂-deficient mice (KO), and 12 days later, lung metastases were analyzed histologically (n=7-10). Shown are mean values ± S.D. (D,E) or S.E.M. (A-C) ; *, *P*<0.05; **, *P*<0.01; ***, *P*<0.001; n.s., not significant.

The examples illustrate the invention:

### Example 1: Role of ATP

Since platelets potentiate endothelial cell retraction induced by tumour cells *in vitro* and *in vivo (21-23),* we explored whether platelets facilitate transmigration of tumour cells through the endothelium. Various mouse and human tumour cells migrated through an endothelial cell layer, and this ability was enhanced by the presence of platelets (Fig. 1A). Since supernatants from platelets stimulated by tumour cells also promoted tumour cell transmigration, whereas supernatants from unstimulated platelets were without effect (Fig. 1 B), we tested whether mediators derived from activated platelets were involved. While inhibition of thromboxane A₂ formation by the cyclooxygenase inhibitor flurbiprofen and blockade of serotonin 5-HT₂ receptors by ketanserin had no effect, degradation of platelet-derived nucleotides such as ATP and ADP using apyrase ablated platelet-dependent facilitation of tumour cell transmigration (Fig. 1 C), suggesting an involvement of nucleotides released from platelets. Consistent with this, tumour cells induced a strong release of ATP from platelet dense granules (Fig. 1 D), and addition of stable analogues of ATP and ADP stimulated tumour cell transmigration *per se* (Fig. 1 E). The interaction of platelets with tumour cells enhanced diffusion of FITC-labeled dextran over an endothelial cell layer and induced a loss of tight interactions of endothelial cells in the monolayer, effects which were both blocked by apyrase (Fig. 1F and G). These data indicate that adenine nucleotides released from dense granules of tumour cellactivated platelets promote an opening of the endothelial barrier and a transmigration of tumour cells through the endothelial layer.

### Example 2: Role of Munc13-4

To study the role of dense granule secretion in platelet-facilitated tumour cell transmigration, we analyzed mice lacking the exocytosis priming protein Munc13-4 which is required for regulated exocytosis and is the predominant Munc13-isoform in platelets *(24, 25).* Munc13-4-deficient mice have normal platelet numbers, platelet integrin αIIbβ3 activation, adhesion to collagen and an unchanged ability to form aggregates with tumour cells *(25)*. Furthermore, thrombin- and tumour cell-induced release of proteins stored in α-granules, such as the chemokine platelet factor 4 (PF4) or von Willebrand factor (vWF), was not affected by Munc13-4 deficiency (Fig. 2A-D). In contrast, Munc13-4-deficient platelets did not release ATP stored in platelet dense granules in response to thrombin or tumour cells (Fig. 2E and F) or U46619 and collagen. Importantly, Munc-1 3-4-deficient platelets also lost the ability to enhance transmigration of tumour cells (Fig. 2G), indicating a role of dense granule secretion but not α-granule secretion in platelet-dependent migration of tumour cells through an endothelial layer.

To test whether platelet dense granule secretion is required for tumour cell metastasis *in vivo,* we analyzed tumour growth and metastasis in wild-type and Munc13-4-deficient mice. While the growth of primary tumours after subcutaneous injection of B16F10 melanoma (B16) and Lewis lung carcinoma 1 tumour cells (LLC1) was indistinguishable between wild-type mice and animals lacking Munc13-4 (Fig. 3A and C), the number of metastases was strongly reduced in Munc13-4-deficient animals (Fig. 3B and D). The effect of Munc13-4-deficiency did not seem to be caused by differences at the primary site of tumour growth or during intravasation since reduced metastasis in Munc13-4-deficient mice was also observed when B16 and LLC1 cells were injected i.v. (Fig. 3E and F). It is unlikely that the inhibition of metastasis in Munc13-4-deficient mice is due to a defective NK cell function which has been shown to involve Munc13-4 *(24)*, as NK cell dysfunction would rather result in increased metastasis *(10, 15)*. Consistent with this, NK cell depletion did not affect the metastasis-reducing effect of Munc-13-4-deficiency.

### Example 3: Role of purinergic receptors

Given the evidence for a role of ATP and ADP in platelet-stimulated tumour cell transmigration and metastasis *in vitro* and *in vivo,* we tested whether ATP/ADP receptors on endothelial cells are involved. Several purinergic receptors activated by ATP/ADP are present on endothelial cells, in particular P2Y1, P2Y2 and P2X4 *(26)*. In the murine endothelial cell line MS1, in primary mouse pulmonary endothelial cells and in human umbilical vein endothelial cells (HUVECs), we found predominant expression of the metabotropic P2Y2 and the ionotropic P2X4 receptors while expression of P2Y1 was low. Neither blockade of P2Y1 nor of P2X4 receptors reduced platelet dependent tumour cell transmigration *in vitro* (Fig. 4A). However, endothelial knockdown of the ATP/ADP-receptor P2Y2, for which no specific antagonist is available, significantly reduced platelet-mediated tumour cell transmigration (Fig. 4B). We therefore analyzed the ability of tumour cells to open the endothelial barrier and to form metastases in mice lacking ATP/ADP-secretion from platelets or in animals deficient in P2Y₂. Within 3 hours after i.v, injection of B16 cells, an opening of the endothelial barrier indicated by increased extravasation of FITC-labeled dextran could be observed in the lungs of wild-type mice, but not in Munc13-4- or P2Y₂- deficient mice (Fig. 4C). Similar to the results in mice lacking Munc13-4, absence of P2Y₂ strongly reduced occurrence of metastases when mice injected with B16 or LLC1 cells were compared to wild-type mice (Fig. 4D and E), indicating that the ATP/ADP-receptor P2Y₂ mediates platelet dependent tumour cell metastasis.

### Example 4: Analysis

The interaction of tumour cells with their microenvironment has been recognized as an important factor in tumour metastasis *(1-3)*. Here we provide evidence that platelet dense granule secretion of adenine nucleotides in the microenvironment of tumour cells is required for efficient tumour cell extravasation and formation of metastasis, and that this involves activation of endothelial purinergic P2Y₂ receptors by ATP/ADP. Thus, besides their role in promoting tumour cell survival in the circulation and inducing a prometastatic phenotype, our study shows that platelets actively enhance tumour cell migration across the endothelial cell layer. This is consistent with the close association of platelets with metastasizing tumour cells during the first hours after vascular arrest *(14, 22)* and with the ability of platelets to increase vascular permeability and to promote extravasation of various other cell types *(23, 27, 28).* These data indicate that platelets promote tumour cell extravasation at early stages after vascular arrest, a critical time period in which the endothelial barrier has to be opened to allow tumour cells access to the underlying basement membrane. Endothelial P2Y₂ receptors activated by platelet-derived adenine nucleotides appear to mediate this process by coupling to the G-proteins G_{q}/G₁₁ *(26)*. Activation of the G_{q}/G₁₁-mediated pathway resulting in an elevation of [Ca²⁺]i and activation of protein kinase C has been shown to induce opening of the endothelial barrier *(29-3 1)* and thereby can facilitate the localized migration of tumour cells through the endothelium. This is also supported by recent observations indicating a role of the endothelial P2Y₂ receptor in promoting transendothelial migration of neutrophils (32).

Despite the evidence for a role of platelets in tumour metastasis, clinical trials have been rare due to concerns that anti-platelet drugs affect normal platelet function leading to bleeding complications *(8, 9)*. The identification of mechanisms involved in platelet-dependent metastasis downstream of platelets provides a basis for new approaches to interfere with platelet-dependent tumour cell dissemination without affecting physiological platelet functions. Given the central role of endothelial P2Y₂ receptors in mediating the prometastatic effects of platelet-derived nucleotides, blockade of P2Y₂ or endothelial downstream signaling pathways may serve as a target to develop novel strategies to prevent tumour cell metastasis.

### Example 5:

### 5.1 Materials

Flurbiprofen and U46619 were from Cayman Chemical, ketanserin, FITC-dextran 70 kDa, 2-methylthio-adenosine-5'-diphosphate (2MeSADP), calcein-AM, bovine serum albumin (essentially fatty acid free), apyrase grade VII and thrombin were from Sigma, fibrillar type I collagen was from Nycomed, adenosine 5'-O-(3-thio)triphosphate (ATPγS) was from Jena Bioscience, carboxyfluorescein diacetate succinimidyl ester (CFSE) and FITC-dextran 70 kDa lysine fixable were from Invitrogen, 5-BDBD and MRS2500 were from Tocris.

### 5.2 Cells

All cells were incubated at 37°C and 5% CO2. Human umbilical vein endothelial cells (HUVECs; Lonza) were grown in EGM2 medium and passages < P6 were used for all experiments. All other cell lines were cultured in the presence of 10% FBS and penicillin/streptomycin each at 100 units/ml in RMPI (B16F10 melanoma cells), DMEM and 2 mM glutamine (Lewis lung carcinoma 1 (LLC1) and Mile Sven 1 (MS1) endothelial cells), DMEM, 2 mM glutamine and 100 µM non-essential amino acids (SH-SY5Y neuroblastoma and CF-PAC1 adenocarcinoma cells).

### 5.3 Platelet preparation

Blood was collected from 2-4 mice or healthy volunteers and was anticoagulated with citrate. Platelet-rich plasma (PRP) was prepared by centrifugation at 150 × g for 7 min. The PRP was centrifuged at 350 × g for 7 min, and the platelet pellet was gently resuspended in HEPES/Tyrode's buffer (TB; 10 mM HEPES/NaOH, pH 7.4, 5.56 mM glucose, 0.3% w/v BSA (essentially fatty acid free), 137 mM NaCl, 12 mM NaHCO3, 2.7 mM KCI, 0.36 mM NaH2PO4, 1 mM MgCl₂). Platelet concentrations were determined by a hemocytometer.

### 5.4 Transwell assay

MS1 cells (8 x 10³ at seeding in 50 µl) or HUVEC (6 x 10³ at seeding in 50 µl) were cultured for 2 days on 96-transwell plates with polyester membranes of 8-µm pore size (Corning) with medium changes every day. For knockdown experiments, 4 x 10⁵ MS1 cells were transfected using Amaxa Nucleofector Kit V with different sets of siRNA against the P2Y₂ receptor (Qiagen) and 1.2 x 10⁴ cells in 50 µl were seeded to the up-per compartment. Where indicated, MS1 cells were pre-incubated with 5-BDBD (1 µM) or MRS2500 (100 nM) for 30 min. Before the experiment, exemplary wells of each condition were visually checked by calcein-AM staining of the endothelial monolayer and only plates with 100% confluency were used. For transmigration, the medium from the upper compartment was removed and 50 µl of a 1:1 mixture of platelets (2 x 10⁸/ml) with calcein-AM-labeled tumour cells (8 x 10⁵/ml) in TB was added to the upper compartment alone or with flurbiprofen (100 nM), ketanserin (3 nM) or apyrase (20 U/ml). ATPγS (10 µM) and 2MeSADP (10 µM) were added in the absence of platelets. For supernatant experiments, human platelets (5 x 10⁷/ml) or mouse platelets (2 x 10⁸/ml) were incubated for 30 min at 37°C alone or with tumour cells (1.6 x 10⁶/ml) and centrifuged at 350 x g for 10 min. The supernatant was mixed 1:1 with TB (for MS1 cells) or cell culture medium (for HUVECs) containing calcein-AM-labeled tumour cells (8 x 10⁵/ml) and 50 µl were added to the upper compartment. In all experiments, 250 µl TB or culture medium were added to the lower compartment, and tumour cells were allowed to transmigrate over night. On the next day, the non-transmigrated tumour cells from the upper compartment were removed and only the transmigrated tumour cells on the lower side of the filter or the bottom of the transwell were imaged (Zeiss Axio Observer.Z1 and Olympus IX81) and quantified with ImageJ (NIH), Each experiment was performed at least 3 times with a minimum of 5 wells per experiment.

### 5.5 Endothelial barrier

The permeability assay was performed as described for the transwell assay with some modifications. Briefly, MS1 cells were cultured on transwell filters and the supernatant of platelets alone or platelets stimulated with tumour cells was mixed 1:1 with TB containing 2 mg/ml 70 kDa FITC-DX of which 50 µl were added to the upper compartment in the absence or presence of apyrase (20 U/ml). 250 µl TB were added to the lower compartment. After 90 min, the amount of passed FITC-DX to the lower compartment was measured (FlexStation3, Molecular Devices). For visualization of changes in the endothelial barrier, MS1 cells were stimulated for 1 h with supernatants from tumour cells alone or from tumour cells after stimulation with platelets in the absence or presence of apyrase (20 U/ml). Cells were further processed for staining and imaging

### 5.6 Immunostaining and Imaging

Cells or tissues were fixed with 4% para-formaldehyde. Tissues were further cryopreserved in 30% sucrose (Sigma), embedded in OCT (Fisher Scientific), and cryosectioned at 16 µm. Cells or sections were stained with rat anti-VE-Cadherin (BD Pharmingen) and Alexa Fluor (AF) 594- (Molecular Probes) or Cy5- (Dianova) conjugated secondary antibodies. DAPI (Sigma) was used to stain cell nuclei, and AF488-conjugated phalloidin (Molecular Probes) to stain F-actin. Images were acquired using a Leica SP5 confocal microscope and analyzed using ImageJ (NIH).

### 5.7 Analysis of platelet function

### ATP release

ATP secretion by platelets was determined using a luciferin/luciferase ATP kit (Enliten, Promega). For this purpose, 10 µl aliquots of supernatant from platelets (1 x 10⁸/ml) treated with agonist or 1 x 10⁶ tumour cells/ml were mixed with 100 µl of ATP reagent, and light emission was measured using a luminometer (Thermo Scientific). Raw data were collected as relative light units integrated over 0.1 s and calibrated using an ATP standard.

### PF4 release

10 µl aliquots of supernatant from platelets (1 x 10⁸/ml) treated with agonist or tumour cells (1 x 10⁶/ml) were assayed in triplicates in a standard mouse PF4 ELISA Kit (Raybiotech) according to manufacturer's instructions. Absorption at 450 nm was measured in a spectrophotometer (Thermo Scientific). Level of PF4 in the samples was calculated by comparison with a PF4 standard dilution series.

### vW factor release

50 µl aliquots of supernatant from platelets (1 x 10⁸/ml) treated with agonist or tumour cells (1 x 10⁶/ml) were assayed in triplicates in an ELISA plate (Nunc) coated with a human vWF capturing antibody (Dako). The samples were analyzed with a secondary HRP-labeled vWF antibody (Dako) and substrate solution containing OPD tablets (Dako) and _{H2O2} (Sigma) according to Dako's general ELISA procedure. Absorption at 490 nm was measured in a spectrophotometer (Thermo Scientific). Level of vWF in the samples were calculated compared to a vWF standard dilution series. *Interaction of tumour cells with CFSE-labeled platelets*

Isolated mouse platelets (1 x 10⁸/ml) were stained with CFSE (Invitrogen) for 30 min and washed in TB. B16 tumour cells resuspended in TB (1 x 10⁶/ml) were mixed 1:1 with platelets and incubated for 30 min on a shaker at 37°C and 200 rpm. Platelet-tumour cell suspensions were analyzed in triplicates by flow cytromentry (FACSCanto; BD Pharmingen). Mean fluorescence intensities of tumour cells were identified by forward/sideward scatter (FSC/SSC) in the FITC channel.

### Flow cytometry

For flow cytometry, washed platelets were adjusted to 1 x 10⁶ platelets/ml with TB, and 25 µl were stimulated with the indicated agonists for 10 min at 37°C. Samples were then stained with a PE-labeled antibody against active mouse integrin αIIbβ3 (GPIIb/IIIa; Emfret) for 20 min at room temperature and directly analyzed by flow cytometry (FACSCanto; BD Pharmingen). Platelets were gated by forward/sideward scatter (FSC/SSC) characteristics and identified by anti-CD41 staining (BD Pharmingen).

### Adhesion to Colla gen

Heparinized blood (final concentration 5 U/ml) of wild-type and Munc13-4-deficient mice was mixed 1:1 with TB and was applied to a flow chamber set-up containing collagencoated coverslips. Before the experiment, coversilps were blocked in 5% BSA for 2 hours at RT, fixed to the flow chamber and rinsed with TB supplemented with heparin (5 U/ml; Braun Melsungen). The transparent flow chamber had a slit depth of 50 µm and was connected to a syringe filled with the diluted blood samples. Perfusion was carried out at room temperature using a pulse-free pump with a wall shear rate of 1000 s⁻¹. Perfusion was stopped after 2 min and chambers were rinsed with TB for 10 min. Coverslips were analyzed by taking 5 randomly chosen brightfield images per coverslip on a stereomicroscope

### (Zeiss Axio Observer Z.1)

### Western blotting

Washed mouse platelets were centrifuged at 350 x g for 7 min, and the pellet was resuspended in Laemmli buffer. The samples were lysed at 90°C for 5 min and the lysate of 5 x 106 platelets per lane was separated on a SDS polyacrylamide gel. Munc13-4 levels were detected using a mouse anti-Munc13-4 antibody (Santa Cruz). An anti-panactin antibody (Acris) was used as loading control.

### Platelet count

100 µl of blood per mouse was taken in EDTA (5 mM final concentration), and a defined concentration of fluorescent particles (Spherotech) was added. Platelets were stained with a FITC-labeled antibody against CD41 (BD Pharmingen) for 30 min at RT. Using flow cytometry, platelets were counted as CD41 positive events relative to the number of fluorescent particles.

### 5.8 Genetic mouse models

Control C57BL/6J animals and P2Y2-deficient mice (B6.129P2-*P2ry2^{tm1Bhk}*/J) were obtained from The Jackson Laboratory (Bar Harbor, ME). The Munc13-4 mutant mouse line *(Unc13d^{Jinx})* was kindly provided by B. Beutler (Department of Genetics, The Scripps Research Institute, La Jolla, USA). All experimental animal procedures were approved by the Regierungspräsidien Darmstadt and Karlsruhe.

### 5.9 Tumour models

B16F10 melanoma cells or LLC1 cells were harvested and resuspended in PBS. Mice (at 8-10 weeks of age) were anesthetized with isoflurane (Abbot Laboratories), and 1 x 10⁶ B16 or LLC1 cells in 100 p l PBS were were injected subcutaneously to their flanks. Mice with B16 primary tumour were sacrificed 19 days and mice with LLC1 tumour 21 days after injection of tumour cells, and primary tumours as well as lung metastases were analyzed. Alternatively, 125 µl of a tumour cell (5 x 104 cells) suspension was injected to the lateral tail vein of mice, and lung metastases were analyzed 12 days later. Animals were perfused with PBS and para-formaldehyde. Primary tumours were removed and weighed. For analysis of pulmonary metastases, the lungs were removed and fixed in para-formaldehyde. Lungs were then cut in 5 µm sections and one section every 200 µm throughout the whole lung was screened histologically, the number of metastases was counted and assigned to the respective size category: small (diameter: <0.1 mm), medium (diameter: 0.1-1.0 mm) or large (diameter: >1.0 mm).

### 5.10 Analysis of endothelial barrier function in vivo

Mice were anesthetized using isoflurane (Abbot Laboratories), and 100 µl of fixable FITC-dextran with a molecular weight of 70 kDa (Invitrogen) in 0.9% saline was injected retroorbitally at a concentration of 15 mg/kg. One min later, 1 x 105 B16 tumour cells in 125 µl PBS were injected via the lateral tail vein. After 3 hours, animals were sacrificed, and lungs were carefully excised. Lungs were fixed in para-formaldehyde, cryopreserved in 30% sucrose and further processed for immunostaining and imaging.

### 5.11 NK cell depletion

NK cells were depleted with an anti-asialo GM1 depleting antibody (Wako). 25 µl of antibody solution diluted 1:4 in 0.9% saline per animal was injected i.v. One day after initial injection of antibody, 5 x 10⁴ B16 tumour cells in PBS were injected in the tail vein of mice. Injection of antibody was repeated every 3.5 days throughout the experiment. Animals were sacrificed on day 12 after tumour cell injection, and the number of lung metastases was determined as described above. NK cell levels were determined via flow cytometry before the first injection of antibody (=basal) and on day 7 and 12 after injection of tumour cells. For this purpose, 50 µl of blood per mouse was taken in EDTA (5 mM final concentration) and NK cells were stained with an APC-labeled antibody against NK1.1 (BD Pharmingen) and a FITC-labeled antibody against CD3e (BD Pharmingen) for 30 min at RT. NK cells were identified as NK1.1 and CD3e positive events and expressed as percent of granulocyte population (identified in a fixed gate in forward/sideward scatter).

### 5.12 Expression analysis

For isolation of mouse primary pulmonary endothelial cells, lungs were minced and digested in 50 U/ml dispase for 1 h at 37°C with shaking (350 rpm). After filtration, the cells were washed in PBS containing 0.5% BSA. Cells were incubated with anti-CD144 antibody-coated (BD Pharmingen) magnetic beads (Invitrogen) for 1 h at room temperature, washed, and isolated with a magnet (invitragen). Cells were grown in DMEM/F12 (Invitrogen) supplemented with 10% FBS, penicillin/streptomycin, and EC growth supplement with heparin (PromoCeli) on fibronectin-coated wells. Cells were harvested, and RNA was isolated with a standard RNA isolation kit (Qiagen) according to manufacturer's instructions. RNA was transcribed using a kit (Roche), and quantitative PCR was performed employing the Universal Probe Library System (Roche) on a Lightcycler 480 instrument (Roche). Primers were designed with the online tool provided by Roche. Relative expression levels were obtained by normalizing with GAPDH and 18S rRNA values, respectively. Sequences for forward (Fw) and Reverse (Rev) primers of mouse and human receptors are depicted from 5' to 3' end. Primers for mouse P2Y receptors were: P2Y1: Fw CTGTGTGGACCCCATTCTTT (SEQ ID NO: 9), Rev TCGGGACAGTCTCCTTCTGA (SEQ ID NO: 10), probe #19; P2Y2: Fw1 TGGTACTGGCCGTCTTCG (SEQ ID NO: 11), Rev1 AGTAGAGGGTGCGCGTGA (SEQ ID NO: 12), probe #4, Fw2 CTATGCCGCCTCAAAACCT (SEQ ID NO: 13), Rev2 CGAAACTGCCAGGTGAAAC (SEQ ID NO: 14), probe #22; P2Y4: Fw CGGCGACTGTATCGACCT (SEQ ID NO: 15), Rev GAGAGAACGGAGCCGAGAA (SEQ ID NO: 16), probe #1 7; P2Y6: Fw GCAGTCTTTGCTGCCACA (SEQ ID NO: 17), Rev GTGGGCTCAGGTCGTAGC (SEQ ID NO: 18), probe #42; P2Y12: Fw TGCTGTACACCGTCCTGTTC (SEQ ID NO: 19), Rev1 AAATCCTCATTGCCAAGCTG (SEQ ID NO: 20), probe #1 07; P2Y13: Fw ATGTGTGAGATGGGGAAAGG (SEQ ID NO: 21), Rev CTGACTACTGTGGTGGTCTTCG (SEQ ID NO: 22), probe #18; P2Y14: Fw TCTTAGAAGACACCCAGTCTTTCC (SEQ ID NO: 23), Rev AAATAGATACGAGTGTTGCTTGGA (SEQ ID NO: 24), probe #69.

Sequences for mouse P2X primers were: P2X1: Fw TGTAAGCCCCTGTCCCATC (SEQ ID NO: 25), Rev TCCTGCAGAGGACATGTGG (SEQ ID NO: 26), probe #1 9; P2X2: Fw GCTCCAGCTCTGCTCTGACT (SEQ ID NO: 27), Rev TTTGTCCCATATGCTGGTCA (SEQ ID NO: 28), probe #77; P2X3: Fw TTTCATCAACCTCCCCTCTG (SEQ ID NO: 29), Rev GACAGTACCCCATCACCCATA (SEQ ID NO: 30), probe #1 00; P2X4: Fw AGAGGAATATCCTCCCCAACAT (SEQ ID NO: 31), Rev GCGCATTATAAATGCACGAC (SEQ ID NO: 32), probe #10; P2X5: Fw CACAGTCATCAACATTGGTTCC (SEQ ID NO: 33), Rev AGGTAGATAAGTACCAGGTCACAGAAG (SEQ ID NO: 34), probe #45; P2X6: Fw AGCTGCGGCTAAAGTTGC (SEQ ID NO: 35), Rev GGCTGCAGAAGCCATGTT (SEQ ID NO: 36), probe #1 8; P2X7: Fw CAGTCACTGGAGGAACTGGAA (SEQ ID NO: 37), Rev CCAAAGGAAACACACCGATT (SEQ ID NO: 38), probe #1 7.

Primer for mouse 18S rRNA: Fw GCAATTATTCCCCATGAACG (SEQ ID NO: 39), Rev GGGACT-TAATCAACGCAAGC (SEQ ID NO: 40), probe #48.

Primer for human P2Y receptors were: P2Y1: Fw ATGTTCTGTGTCCCCTTGGT (SEQ ID NO: 41), RevAAATCAAAGCTCTCACAATTAATCC (SEQ iD NO: 42), probe #26; P2Y2: Fw TAACCTGCCACGACACCTC (SEQ ID NO: 43), Rev CTGAGCTGTAGGCCACGAA (SEQ ID NO: 44), probe #41; P2Y4: Fw CATCACCCGCACCATTTACT (SEQ ID NO: 45), Rev TTCAGTACTCGGCAGTCAGC (SEQ ID NO: 46), probe #64; P2Y6: Fw CTGCCCACAGCCATCTTC (SEQ ID NO: 47), Rev GCTGAGGTCATAGCAGACAGTG (SEQ ID NO: 48), probe #42; P2Y11: Fw GTTGGTGGCCAGTGGTGT (SEQ ID NO: 49), Rev CCGCATGATGTGGTAGGG (SEQ ID NO: 50), probe #1 7; P2Y12: Fw CATGATTCTGACTTTTCCATTCA (SEQ ID NO: 51), Rev TGACACACAAAAGTTCTCAGTGG (SEQ ID NO: 52), probe #48; P2Y13: Fw TTTTCTTGACCGGCATCC (SEQ ID NO: 53), Rev AGCTGGGGATGTGAACAAAC (SEQ ID NO: 54), probe #21; P2Y14: Fw CTTGCGGTATATGAAAGAATTCAC (SEQ ID NO: 55), Rev AAGAAA-TAAATAATAGGGTCCAAGCA (SEQ ID NO: 56), probe #84.

Primer for human GAPDH: Fw GCATCCTGGGCTACACTGA (SEQ ID NO: 57), Rev CCAGCGTCAAAGGTGGAG (SEQ ID NO: 58), probe #82.

### 5.13 Statistical analysis

If not stated otherwise, one representative of at least 3 independently performed experiments is shown. All raw data was analyzed with Student's *t*-test. Depicted are mean values ± S.D. or ± S.E.M. as indicated in the figure legends. Statistical significance was defined as *, *P*<0.05; **, *P*<0.01; ***, *P*<0.001; n.s., not significant.

### References

1. J. A. Joyce, J. W. Pollard, Nat. ReV. Cancer 9, 239 (2009).
2. C. L. Chaffer, R. A. Weinberg, Science 331, 1559 (2011).
3. D. X. Nguyen, P. D. Bos, J. Massague, Nat. Rev. Cancer 9, 274 (2009).
4. E. Pearlstein, C. Ambrogio, S. Karpatkin, Cancer Res. 44, 3884 (1984).
5. E. Camerer et al., Blood 104, 397 (2004).
6. K. V. Honn, D. G. Tang, Y. Q. Chen, Semin. Thromb. Hemost. 18, 392 (1992).
7. S. Jain, J. Harris, J. Ware, Arterioscler. Thromb. Vasc. Biol. 30, 2362 (2010).
8. L. Erpenbeck, M. P. Schon, Blood 115, 3427 (2010).
9. L. J. Gay, B. Felding-Habermann, Nat. Rev. Cancer 11, 123 (2011).
10. J. S. Palumbo et al., Blood 105, 178 (2005).
11. L. Borsig et al., Proc. Natl. Acad. Sci. U. S. A. 98, 3352 (2001).
12. W. Ruf, B. M. Mueller, Semin. Thromb. Hemost. 32 Suppl 1, 61 (2006).
13. M. L. Nierodzik, S. Karpatkin, Cancer Cell 10, 355 (2006).
14. J. H. Im et al., Cancer Res. 64, 8613 (2004).
15. B. Nieswandt, M. Hafner, B. Echtenacher, D. N. Mannel, Cancer Res. 59, 1295 (1999).
16. J. E. Italiano, Jr. et al., Blood 111, 1227 (2008).
17. A. Boucharaba et al., J. Clin. Invest. 114, 1714 (2004).
18. M. Labelle, S. Begum, R. O. Hynes, Cancer Cell 20, 576 (2011).
19. G. L. Reed, Semin. Thromb. Hemost. 30, 441 (2004).
20. Q. Ren, S. Ye, S. W. Whiteheart, Curr. Opin. Hematol, 15, 537 (2008).
21. R. H. Kramer, G. L. Nicolson, Proc. Natl. Acad. Sci. U. S. A. 76, 5704 (1979).
22. J. D. Crissman, J. S. Hatfield, D. G. Menter, B. Sloane, K. V. Honn, Cancer Res. 48, 4065 (1988).
23. K. V. Honn et al., Exp. Cell Res. 210, 1 (1994).
24. K. Crozat et al., J. Exp. Med. 204, 853 (2007).
25. Q. Ren et al., Blood 116, 869 (2010).
26. M. P. Abbracchio et al., Pharmaco/. Rev. 58, 281 (2006).
27. T. G. Diacovo, K. D. Puri, R. A. Warnock, T. A. Springer, U. H. von Andrian, Science 273, 252 (1996).
28. B. Petri et al., Blood 116, 4712 (2010).
29. H. Korhonen et al., J. Exp. Med. 206, 411 (2009).
30. J. Gavard, J. S. Gutkind, J. Biol. Chem., (2008).
31. N. Knezevic, M. Tauseef, T. Thennes, D. Mehta, J. Exp. Med. 206, 2761 (2009).
32. F. Kukulski et al., Mol. Immunol. 47, 991 (2010).

## Claims

1. An inhibitor of the P2Y2 receptor or an inhibitor of the P2Y2 receptor signaling pathway for use in preventing the metastasis of tumours or as a lead compound for developing a drug for preventing the metastasis of tumours.

2. The inhibitor according to claim 1, wherein the inhibitor is an antibody or a fragment or derivative thereof, a siRNA, a shRNA, a miRNA, a ribozyme, an aptamer, an antisense nucleic acid molecule, a small molecule or a modified version of these inhibitors.

3. The inhibitor according to claim 1 or 2, wherein the inhibitor of the P2Y2 receptor signaling pathway is an inhibitor of G-proteins G_{q}/G₁₁, phospholipase Cβ and/or protein kinase C (PKC).

4. The inhibitor according to any one of claims 1 to 3, wherein the inhibitor of the P2Y2 receptor is a siRNA with the nucleic acid sequence of any one of SEQ ID NOs: 1 to 8.

5. The inhibitor according to any one of claims 1 to 4 having admixed thereto or being associated in a separate container with a further pharmaceutically active agent.

6. A method of preventing the metastasis of tumours comprising administering a pharmaceutically effective amount of an inhibitor of the P2Y2 receptor or an inhibitor of the P2Y2 receptor signaling pathway to a subject in need thereof.

7. The method according to claim 6, wherein the inhibitor is an antibody or a fragment or derivative thereof, a siRNA, a shRNA, a miRNA, a ribozyme, an aptamer, an antisense nucleic acid molecule, a small molecule or a modified version of these inhibitors.

8. The method according to claim 6 or 7 wherein the inhibitor of the P2Y2 receptor signaling pathway is an inhibitor of G-proteins G_{q}/G₁₁, phospholipase Cβ and/or protein kinase C (PKC).

9. The method according to any one of claims 6 to 8, wherein the inhibitor of the P2Y2 receptor is a siRNA with the nucleic acid sequence of any one of SEQ ID NO: 1 to 8.

10. The method according to any one of claims 6 to 9, further comprising administering a further pharmaceutically active agent.


SEQUENCE LISTING

<110>  Max-Planck-Gesellschaft zur Förderung der Wissenschaften
       e.V.

<120>  Novel therapeutic target for the prevention of tumour metastasis

<130>  U1289 EP

<160>  58

<170>  PatentIn version 3.5

<210>  1
<211>  21
<212>  DNA
<213>  Artificial sequence

<220>
<223>  siRNA Mm_P2ry2_6

<400>  1
caggtgggaa ccataactta t                                                21


<210>  2
<211>  21
<212>  DNA
<213>  Artificial sequence

<220>
<223>  siRNA Mm_P2ry2_7

<400>  2
cagggagagc ccattacgtg a                                                21


<210>  3
<211>  21
<212>  DNA
<213>  Artificial sequence

<220>
<223>  siRNA Mm_P2ry2_2

<400>  3
atccaacaac tggttcttaa a                                                21


<210>  4
<211>  21
<212>  DNA
<213>  Artificial sequence

<220>
<223>  siRNA Mm_P2ry2_5

<400>  4
ctggcaaatg tcaaaccatt a                                                21


<210>  5
<211>  21
<212>  DNA
<213>  Artificial sequence

<220>
<223>  siRNA Hs_P2RY2_4

<400>  5
gaggctgtaa cttatactaa a                                                21


<210>  6
<211>  21
<212>  DNA
<213>  Artificial sequence

<220>
<223>  siRNA Hs_P2RY2_6

<400>  6
tgccgtcatc cttgtctgtt a                                                21


<210>  7
<211>  21
<212>  DNA
<213>  Artificial sequence

<220>
<223>  siRNA Hs_P2RY2_1

<400>  7
ctggaatgcg tccaccacat a                                                21


<210>  8
<211>  21
<212>  DNA
<213>  Artificial sequence

<220>
<223>  siRNA Hs_P2RY2_2

<400>  8
caccggtgtc tgggcgtctt a                                                21


<210>  9
<211>  20
<212>  DNA
<213>  Artificial sequence

<220>
<223>  mouse P2Y1 Fw

<400>  9
ctgtgtggac cccattcttt                                                  20


<210>  10
<211>  20
<212>  DNA
<213>  Artificial sequence

<220>
<223>  mouse P2Y1 Rev

<400>  10

tcgggacagt ctccttctga                                                    20

<210>  11
<211>  18
<212>  DNA
<213>  Artificial sequence

<220>
<223>  mouse P2Y2 Fw1

<400>  11
tggtactggc cgtcttcg                                                      18

<210>  12
<211>  14
<212>  DNA
<213>  Artificial sequence

<220>
<223>  mouse P2Y2 Rev1

<400>  12
gagggtgcgc gtga                                                          14

<210>  13
<211>  19
<212>  DNA
<213>  Artificial sequence

<220>
<223>  mouse P2Y2 Fw2

<400>  13
ctatgccgcc tcaaaacct                                                     19

<210>  14
<211>  19
<212>  DNA
<213>  Artificial sequence

<220>
<223>  mouse P2Y2 Rev2

<400>  14
cgaaactgcc aggtgaaac                                                     19

<210>  15
<211>  18
<212>  DNA
<213>  Artificial sequence

<220>
<223>  mouse P2Y4 Fw

<400>  15
cggcgactgt atcgacct                                                      18

<210>  16
<211>  19

<212>  DNA
<213>  Artificial sequence

<220>
<223>  mouse P2Y4 Rev

<400>  16
gagagaacgg agccgagaa                                                    19


<210>  17
<211>  18
<212>  DNA
<213>  Artificial sequence

<220>
<223>  mouse P2Y6 Fw

<400>  17
gcagtctttg ctgccaca                                                     18


<210>  18
<211>  18
<212>  DNA
<213>  Artificial sequence

<220>
<223>  mouse P2Y6 Rev

<400>  18
gtgggctcag gtcgtagc                                                     18


<210>  19
<211>  20
<212>  DNA
<213>  Artificial sequence

<220>
<223>  mouse P2Y12 Fw

<400>  19
tgctgtacac cgtcctgttc                                                   20


<210>  20
<211>  20
<212>  DNA
<213>  Artificial sequence

<220>
<223>  mouse P2Y12 Rev1

<400>  20
aaatcctcat tgccaagctg                                                   20


<210>  21
<211>  20
<212>  DNA
<213>  Artificial sequence

<220>
<223>  mouse P2Y13 Fw

<400>  21
atgtgtgaga tggggaaagg                                                    20

<210>  22
<211>  22
<212>  DNA
<213>  Artificial sequence

<220>
<223>  mouse P2Y13 Rev

<400>  22
ctgactactg tggtggtctt cg                                                 22

<210>  23
<211>  24
<212>  DNA
<213>  Artificial sequence

<220>
<223>  mouse P2Y14 Fw

<400>  23
tcttagaaga cacccagtct ttcc                                               24

<210>  24
<211>  24
<212>  DNA
<213>  Artificial sequence

<220>
<223>  mouse P2Y14 Rev

<400>  24
aaatagatac gagtgttgct tgga                                               24

<210>  25
<211>  19
<212>  DNA
<213>  Artificial sequence

<220>
<223>  mouse P2X1 Fw

<400>  25
tgtaagcccc tgtcccatc                                                     19

<210>  26
<211>  19
<212>  DNA
<213>  Artificial sequence

<220>
<223>  mouse P2X1 Rev

<400>  26
tcctgcagag gacatgtgg                                                     19

<210>  27
<211>  20
<212>  DNA
<213>  Artificial sequence

<220>
<223>  mouse P2X2 Fw

<400>  27
gctccagctc tgctctgact                                                    20


<210>  28
<211>  20
<212>  DNA
<213>  Artificial sequence

<220>
<223>  mouse P2X2 Rev

<400>  28
tttgtcccat atgctggtca                                                    20


<210>  29
<211>  20
<212>  DNA
<213>  Artificial sequence

<220>
<223>  mouse P2X3 Fw

<400>  29
tttcatcaac ctcccctctg                                                    20


<210>  30
<211>  21
<212>  DNA
<213>  Artificial sequence

<220>
<223>  mouse P2X3 Rev

<400>  30
gacagtaccc catcacccat a                                                  21


<210>  31
<211>  22
<212>  DNA
<213>  Artificial sequence

<220>
<223>  mouse P2X4 Fw

<400>  31
agaggaatat cctccccaac at                                                 22


<210>  32
<211>  20
<212>  DNA
<213>  Artificial sequence

<220>
<223>  mouse P2X4 Rev

<400>  32
gcgcattata aatgcacgac                                                    20


<210>  33
<211>  22
<212>  DNA
<213>  Artificial sequence

<220>
<223>  mouse P2X5 Fw

<400>  33
cacagtcatc aacattggtt cc                                                 22


<210>  34
<211>  27
<212>  DNA
<213>  Artificial sequence

<220>
<223>  mouse P2X5 Rev

<400>  34
aggtagataa gtaccaggtc acagaag                                            27


<210>  35
<211>  18
<212>  DNA
<213>  Artificial sequence

<220>
<223>  mouse P2X6 Fw

<400>  35
agctgcggct aaagttgc                                                      18


<210>  36
<211>  18
<212>  DNA
<213>  Artificial sequence

<220>
<223>  mouse P2X6 Rev

<400>  36
ggctgcagaa gccatgtt                                                      18


<210>  37
<211>  21
<212>  DNA
<213>  Artificial sequence

<220>
<223>  mouse P2X7 Fw

<400>  37
cagtcactgg aggaactgga a                                                  21

<210>  38
<211>  20
<212>  DNA
<213>  Artificial sequence

<220>
<223>  mouse P2X7 Rev

<400>  38
ccaaaggaaa cacaccgatt                                                    20


<210>  39
<211>  20
<212>  DNA
<213>  Artificial sequence

<220>
<223>  Primer for mouse 18S rRNA Fw

<400>  39
gcaattattc cccatgaacg                                                    20


<210>  40
<211>  20
<212>  DNA
<213>  Artificial sequence

<220>
<223>  Primer for mouse 18S rRNA Rev

<400>  40
gggacttaat caacgcaagc                                                    20


<210>  41
<211>  20
<212>  DNA
<213>  Artificial sequence

<220>
<223>  human P2Y1 Fw

<400>  41
atgttctgtg tccccttggt                                                    20


<210>  42
<211>  25
<212>  DNA
<213>  Artificial sequence

<220>
<223>  human P2Y1 Rev

<400>  42
aaatcaaagc tctcacaatt aatcc                                              25


<210>  43
<211>  19
<212>  DNA

<213>  Artificial sequence

<220>
<223>  human P2Y2 Fw

<400>  43
taacctgcca cgacacctc                                                    19


<210>  44
<211>  19
<212>  DNA
<213>  Artificial sequence

<220>
<223>  human P2Y2 Rev

<400>  44
ctgagctgta ggccacgaa                                                    19


<210>  45
<211>  20
<212>  DNA
<213>  Artificial sequence

<220>
<223>  human P2Y4 Fw

<400>  45
catcacccgc accatttact                                                   20


<210>  46
<211>  20
<212>  DNA
<213>  Artificial sequence

<220>
<223>  human P2Y4 Rev

<400>  46
ttcagtactc ggcagtcagc                                                   20


<210>  47
<211>  18
<212>  DNA
<213>  Artificial sequence

<220>
<223>  human P2Y6 Fw

<400>  47
ctgcccacag ccatcttc                                                     18


<210>  48
<211>  22
<212>  DNA
<213>  Artificial sequence

<220>
<223>  human P2Y6 Rev

<400>  48
gctgaggtca tagcagacag tg                                                22

<210>  49
<211>  18
<212>  DNA
<213>  Artificial sequence

<220>
<223>  human P2Y11 Fw

<400>  49
gttggtggcc agtggtgt                                                     18

<210>  50
<211>  18
<212>  DNA
<213>  Artificial sequence

<220>
<223>  human P2Y11 Rev

<400>  50
ccgcatgatg tggtaggg                                                     18

<210>  51
<211>  23
<212>  DNA
<213>  Artificial sequence

<220>
<223>  human P2Y12 Fw

<400>  51
catgattctg acttttccat tca                                               23

<210>  52
<211>  23
<212>  DNA
<213>  Artificial sequence

<220>
<223>  human P2Y12 Rev

<400>  52
tgacacacaa aagttctcag tgg                                               23

<210>  53
<211>  18
<212>  DNA
<213>  Artificial sequence

<220>
<223>  human P2Y13 Fw

<400>  53
ttttcttgac cggcatcc                                                     18

<210>  54

<211>  20
<212>  DNA
<213>  Artificial sequence

<220>
<223>  human P2Y13 Rev

<400>  54
agctggggat gtgaacaaac                                                    20


<210>  55
<211>  24
<212>  DNA
<213>  Artificial sequence

<220>
<223>  human P2Y14 Fw

<400>  55
cttgcggtat atgaaagaat tcac                                               24


<210>  56
<211>  26
<212>  DNA
<213>  Artificial sequence

<220>
<223>  human P2Y14 Rev

<400>  56
aagaaataaa taatagggtc caagca                                             26


<210>  57
<211>  19
<212>  DNA
<213>  Artificial sequence

<220>
<223>  Primer for human GAPDH Fw

<400>  57
gcatcctggg ctacactga                                                     19


<210>  58
<211>  18
<212>  DNA
<213>  Artificial sequence

<220>
<223>  Primer for human GAPDH Rev

<400>  58
ccagcgtcaa aggtggag                                                      18